# EUROPEAN PATENT SPECIFICATION

(11) **EP 4 125 723 B1**
(45) Date of publication and mention of the grant of the patent: **04.09.2024**
(21) Application number: 20935619.5
(22) Date of filing: 13.11.2020
(51) Int. Cl.: A61F 2/24, A61B 17/122, A61F 2/95

(54) **MITRAL VALVE REPAIR DEVICE AND CONTROL HANDLE THEREOF**
MITRALKLAPPENREPARATURVORRICHTUNG UND STEUERGRIFF DAFÜR
DISPOSITIF DE RÉPARATION DE VALVULE MITRALE ET SA POIGNÉE DE COMMANDE

(30) Priority: 11.05.2020 CN 202010390972; 23.06.2020 CN 202010581915
(43) Date of publication of application: 08.02.2023
(73) Proprietor: Shanghai Newmed Medical Co., Ltd., Shanghai 201321 (CN)
(72) Inventor: WANG, Haishan, Shanghai 201321 (CN); YU, Qifeng, Shanghai 201321 (CN); QIN, Tao, Shanghai 201321 (CN)
(74) Representative: Wang, Bo
(86) International application number: PCT/CN2020/128544
(87) International publication number: WO 2021/227412

(56) References cited:
- WO-A1-2013/039810
- CN-A- 106 214 202
- CN-A- 110 536 656
- CN-A- 110 537 946
- CN-A- 111 437 070
- CN-A- 111 437 071
- CN-A- 111 437 072
- CN-A- 111 437 073
- CN-A- 111 467 083
- CN-A- 111 588 516
- CN-U- 212 308 127
- CN-U- 212 308 128
- CN-U- 212 308 129
- US-A1- 2017 143 330
- US-A1- 2019 117 425
- US-A1- 2019 209 324

## Description

### CROSS-REFERENCE TO RELATED APPLICATION

This application claims priority of Chinese Patent Application No. 202010390972.1 filed on May 11, 2020 and Chinese Patent Application No. 202010581915.1 filed on June 23, 2020.

### TECHNICAL FIELD

The present disclosure relates to a medical device, and in particular to a mitral valve repair device and a control handle thereof.

### BACKGROUND

Valves are membrane-like structures that can be opened and closed in organs of humans or some animals. For example, there are four valves in the human's heart, including the aortic valve, the pulmonary valve, the mitral valve, and the tricuspid valve. Taking the human's mitral valve as an example, the mitral valve is located between the left atrium and the left ventricle. When the left ventricle contracts, the mitral valve, acting as a check valve, tightly closes an atrioventricular opening to prevent blood from flowing back from the left ventricle to the left atrium. However, if the mitral valve is diseased, it may be difficult for the mitral valve to be closed when the left ventricle contracts, causing the left atrium to receive a large amount of reflux blood. This may lead to a sharp rise in pressures of the left atrium and the pulmonary vein, an increase in the load of the left ventricular diastolic volume, and further lead to a series of pathological changes such as left ventricular enlargement and pulmonary hypertension. Eventually, it may result in clinical manifestations such as heart failure, arrhythmia, etc., which may be life-threatening in severe cases. A mitral valve repair device can be used to repair the diseased mitral valve, for example, clamp valves of the mitral valve, so that one large hole formed by the valve leaflets changes to two small holes, and thus a regurgitation area is reduced, thereby effectively preventing mitral regurgitation. Similarly, the mitral valve repair device can also be applied to the repair of the heart's tricuspid valve and other valves, achieving the same effect of reducing the regurgitation area by clamping valve leaflets on both sides. The mitral valve repair device may include a control handle configured to control the tissue clamping device to perform valve repair. WO2013/039810A1, filed on March 21, 2013, introduces devices, systems and methods for tissue approximation and repair at treatment sites, in which, many of the devices and systems are adapted to be reversible and removable from the patient at any point without interference with or trauma to internal tissues. US 2019/117425 A1, field on April 25, 2019, discloses a delivery system for delivering a prosthesis includes a sheath, a slide shaft having a threaded outer surface, and a handle, the handle including a resilient cover to provide the internal spring assembly with a biased or nominal operational position. It is desirable to provide a mitral valve repair device and a control handle thereof with improved efficiency and success rate of mitral valve repair and convenient operation of mitral valve repair.

### SUMMARY

The invention is set out in the appended set of claims.

### BRIEF DESCRIPTION OF THE DRAWINGS

The present disclosure is further described in terms of exemplary embodiments. These exemplary embodiments are described in detail with reference to the drawings. It should be noted that the drawings are not to scale. These embodiments are nonlimiting exemplary embodiments, in which like reference numerals represent similar structures throughout the several views of the drawings, and wherein:
FIG. 1 is a structural schematic diagram illustrating a mitral valve repair device according to some embodiments of the present disclosure;
FIG. 2 is a structural schematic diagram illustrating a tissue clamping device and a delivery connection member according to some embodiments of the present disclosure;
FIG. 3 is a structural schematic diagram illustrating an outer clamp arm of the tissue clamping device in an opened state according to some embodiments of the present disclosure;
FIG. 4 is a structural schematic diagram illustrating a delivery connection member according to some embodiments of the present disclosure;
FIG. 5 is a structural schematic diagram illustrating a tissue clamping device in a closed state according to another embodiment of the present disclosure;
FIG. 6 is a structural schematic diagram illustrating the tissue clamping device in an opened state according to another embodiment of the present disclosure;
FIG. 7 is a structural schematic diagram illustrating a flexible tube from a front view according to some embodiments of the present disclosure;
FIG. 8 is a structural schematic diagram illustrating the flexible tube from a rear view according to some embodiments of the present disclosure;
FIG. 9 is a structural schematic diagram illustrating different parts of the flexible tube according to some embodiments of the present disclosure;
FIG. 10 is a structural schematic diagram illustrating different parts of a flexible tube control mechanism according to some embodiments of the present disclosure;
FIG. 11 is a partial sectional diagram illustrating an outer clamp arm control mechanism according to some embodiments of the present disclosure;
FIG. 12 is a structural schematic diagram illustrating a sleeve according to some embodiments of the present disclosure;
FIG. 13 is a structural schematic diagram illustrating a sliding part and a protective cover according to some embodiments of the present disclosure;
FIG. 14 is a structural schematic diagram illustrating an outer clamp arm control mechanism according to another embodiment of the present disclosure;
FIG. 15 is a schematic diagram illustrating an internal structure of the outer clamp arm control mechanism according to another embodiment of the present disclosure;
FIG. 16 is a structural schematic diagram illustrating a threaded engagement mechanism according to another embodiment of the present disclosure;
FIG. 17 is a structural schematic diagram illustrating an engagement member of the threaded engagement mechanism according to another embodiment of the present disclosure;
FIG. 18 is a partial sectional diagram illustrating a sliding part according to another embodiment of the present disclosure;
FIG. 19 is a structural schematic diagram illustrating different parts of the sliding part according to another embodiment of the present disclosure;
FIG. 20 is a structural schematic diagram illustrating an outer clamp arm control mechanism which can indicate an opening angle of the outer clamp arm according to some embodiments of the present disclosure;
FIG. 21 is a schematic diagram illustrating a first indicating device according to some embodiments of the present disclosure;
FIG. 22 is a schematic diagram illustrating a first prompting device according to some embodiments of the present disclosure;
FIG. 23 is a partial sectional diagram illustrating an inner clamp arm control mechanism according to some embodiments of the present disclosure;
FIG. 24 is a structural schematic diagram illustrating a housing according to some embodiments of the present disclosure;
FIG. 25 is a structural schematic diagram illustrating a second control part according to some embodiments of the present disclosure;
FIG. 26 is a structural schematic diagram illustrating the second control part and a locking mechanism at a first perspective according to some embodiments of the present disclosure;
FIG. 27 is a structural schematic diagram illustrating the second control part and the locking mechanism at a second perspective according to some embodiments of the present disclosure;
FIG. 28 is a structural schematic diagram illustrating an inner clamp arm control mechanism which can indicate an opening angle of the inner clamp arm according to some embodiments of the present disclosure;
FIG. 29 is a schematic diagram illustrating a second indicating device according to some embodiments of the present disclosure;
FIG. 30 is a structural schematic diagram illustrating an inner clamp arm control mechanism according to another embodiment of the present disclosure;
FIG. 31 is a partial structural diagram illustrating the inner clamp arm control mechanism according to another embodiment of the present disclosure;
FIG. 32 is a structural schematic diagram illustrating a second control part according to another embodiment of the present disclosure;
FIG. 33 is a partial sectional diagram illustrating the inner clamp arm control mechanism according to another embodiment of the present disclosure;
FIG. 34 is a schematic structural diagram illustrating an outer clamp arm control mechanism according to another embodiment of the present disclosure;
FIG. 35 is a partial structural diagram illustrating an outer clamp arm control mechanism according to another embodiment of the present disclosure;
FIG. 36 is a structural schematic diagram illustrating a sliding part according to another embodiment of the present disclosure;
FIG. 37 is a structural schematic diagram illustrating a control handle according to another embodiment of the present disclosure; and
FIG. 38 is a structural schematic diagram illustrating a guiding block according to another embodiment of the present disclosure.

In the drawings: 100-mitral valve repair device; 200-tissue clamping device; 210-inner clamp arm; 211-first inner clamp arm; 213-second inner clamp arm; 215-barb; 220-outer clamp arm; 221-first outer clamp arm; 223-second outer clamp arm; 230-fixing member; 240-supporting part; 250-connecting member; 260-outer clamping plate; 261-first outer clamping plate; 263-second outer clamping plate; 270-connection pipe; 280-elastic locking member; 300-control handle; 400-outer clamp arm control mechanism; 410-sleeve; 412-first sliding groove; 420-first control part; 421-connection groove; 429-connection block; 430-sliding part; 431-outer cover; 433-middle tube; 434-opening; 435-inner pipe; 437-fixture block; 438-locking groove; 439-locking tooth; 440-driving rod; 450-thread engagement mechanism; 451-manipulation button; 453-first elastic member; 455-engagement member; 456-pressing part; 457-engagement part; 460-fixing block; 470-protective cover; 480-boss; 500-inner clamp arm control mechanism; 510-housing; 511-second sliding groove; 511-1-first sub-sliding groove; 511-2-second sub-sliding groove; 520-second control part; 520-1-first sub-control part; 520-2-second sub-control part; 521-duct; 522-connecting rod; 522-1-first sub-connecting rod; 522-2-second sub-connecting rod; 523-end cover; 523-1-first sub-end cover; 523-2-second sub-end cover; 524-control button; 525-protrusion block; 526-connecting body; 527-groove; 530-locking mechanism; 531-second elastic member; 533-locking button; 535-locking block; 537-tooth-shaped connecting part; 540-guide block; 541-first intersecting hole; 542-second intersecting hole; 600-delivery tube; 610-flexible tube; 612-inner core; 614-outer pipe; 616-notch; 620-flexible tube control mechanism; 621-screw; 622-rotating part; 623-traction part; 625-traction rope; 627-threaded traction block; 629-bending indication device; 700-delivery connection member; 710-main body; 720-first connection piece; 730-second connection piece; 740-fixed supporting rod; 810-first indicating device; 811-angle mark; 821-first sensor; 823-processor; 825-display; 830-first prompting device; 831-first contact part; 833-second contact part; 835-speaker; 910-second indicating device; 911-angle mark; 921-second sensor; 923-processor; 925-display.

### DETAILED DESCRIPTION

The terminology used herein is to describe particular example embodiments only and is not intended to be limiting. As used herein, the singular forms "a," "an," and "the" may be intended to include the plural forms as well, unless the context clearly indicates otherwise. It will be further understood that the terms "comprises," "comprising," "includes," and/or "including" when used in this specification, specify the presence of stated features, integers, steps, operations, elements, and/or components, but do not preclude the presence or addition of one or more other features, integers, steps, operations, elements, components, and/or groups thereof.

These and other features, and characteristics of the present disclosure, as well as the methods of operation and functions of the related elements of structure and the combination of parts and economies of manufacture, may become more apparent upon consideration of the following description with reference to the accompanying drawing(s), all of which form a part of this specification. It is to be expressly understood, however, that the drawing(s) is for the purpose of illustration and description only and are not intended to limit the scope of the present disclosure. It is understood that the drawings are not to scale.

The present disclosure relates to a mitral valve repair device and a control handle thereof. The mitral valve repair device may be configured to repair a mitral valve or other valves (such as a tricuspid valve). The mitral valve repair device includes a tissue clamping device and the control handle. The tissue clamping device may be configured to clamp a valve to repair it. The control handle may be configured to transport and control the tissue clamping device. In some embodiments, the tissue clamping device may reach a predetermined position through multiple paths. For example, the tissue clamping device may be transported to the mitral valve via the femoral vein, inferior vena cava, right atrium, and left atrium to repair the mitral valve. As another example, the tissue clamping device may be transported to the mitral valve via the left atrial appendage and the left atrium to repair the mitral valve.

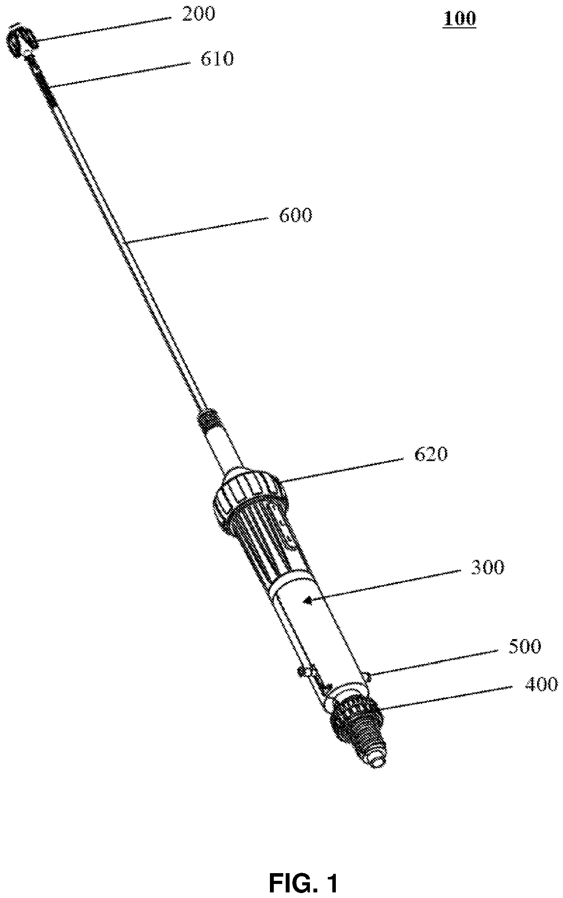

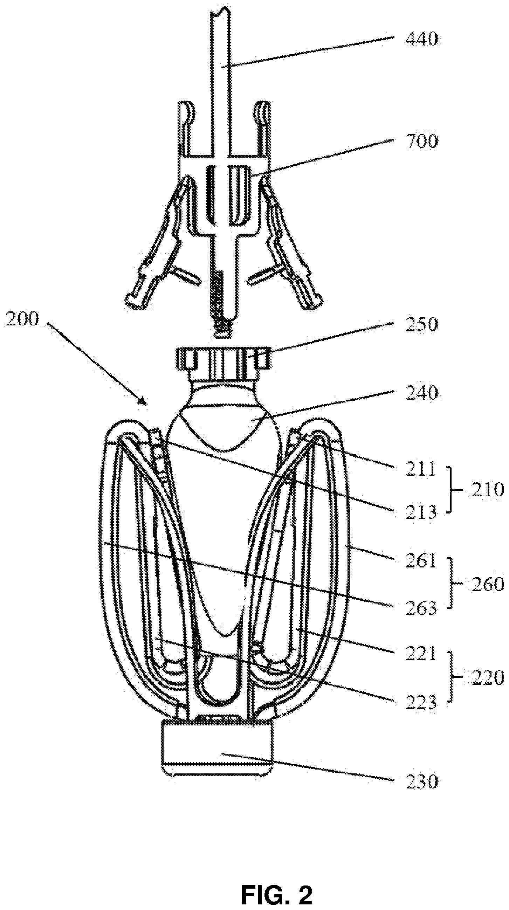

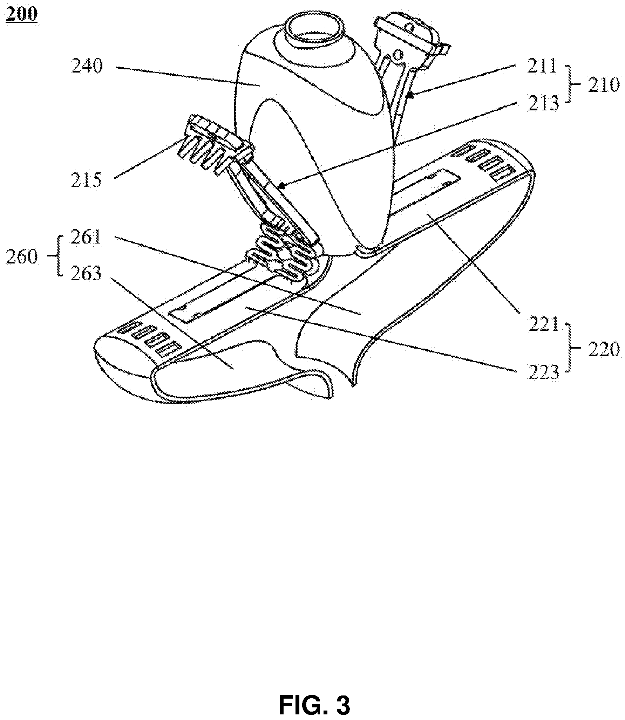

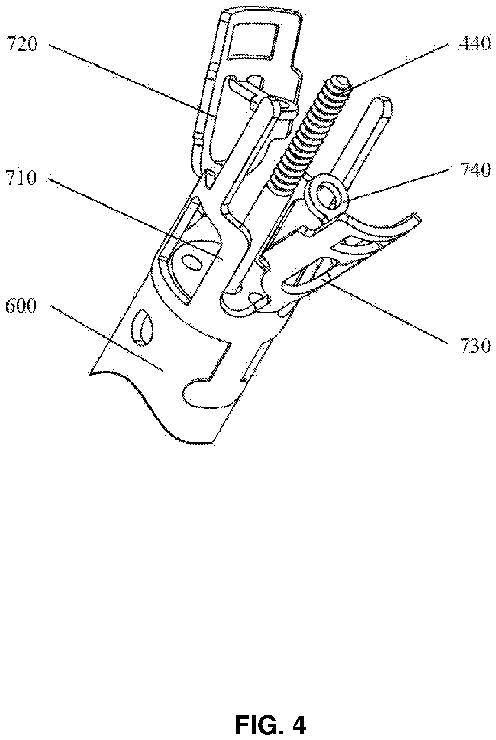

FIG. 1 is a structural schematic diagram illustrating a mitral valve repair device according to some embodiments of the present disclosure. FIG. 2 is a structural schematic diagram illustrating a tissue clamping device and a delivery connection member according to some embodiments of the present disclosure. FIG. 3 is a structural schematic diagram illustrating an outer clamp arm of the tissue clamping device in an opened state according to some embodiments of the present disclosure. FIG. 4 is a structural schematic diagram illustrating a delivery connection member according to some embodiments of the present disclosure. As shown in FIGs. 1 to 4, the mitral valve repair device 100 includes a tissue clamping device 200 and a control handle 300. The tissue clamping device 200 includes an outer clamp arm 220 and an inner clamp arm 210. The control handle 300 includes an outer clamp arm control mechanism 400 and an inner clamp arm control mechanism 500. The outer clamp arm control mechanism 400 is configured to control the opening and closing of the outer clamp arm 220 of the tissue clamping device 200. The inner clamp arm control mechanism 500 is configured to control the opening and closing of the inner clamp arm 210 relative to the outer clamp arm 220. A valve (such as a mitral valve) may be clamped between the outer clamp arm 220 and the inner clamp arm 210 by controlling the opening and closing of the outer clamp arm 220 and the inner clamp arm 210 via the control handle 300.

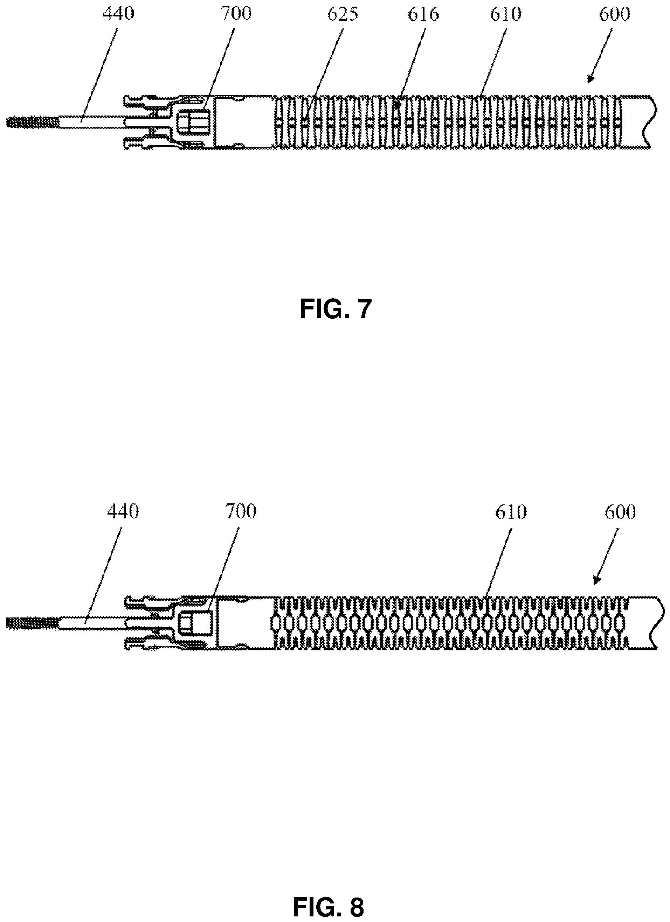

In some embodiments, the mitral valve repair device 100 may include a delivery tube 600. The tissue clamping device 200 may be connected to the control handle 300 via the delivery tube 600. In some embodiments, the delivery tube 600 may be flexible (for example, the delivery tube 600 is made of an elastic material). The control handle 300 may transport the tissue clamping device 200 to the mitral valve through the femoral vein, inferior vena cava, right atrium, or left atrium by the flexible delivery tube 600. In some embodiments, the delivery tube 600 may be inflexible (for example, the delivery tube 600 is made of a rigid material). In such cases, the control handle 300 may transport the tissue clamping device 200 to the mitral valve through the left atrial appendage or the left atrium by the inflexible delivery tube 600. In some embodiments, the delivery tube 600 may include a flexible tube 610 in order to better control the tissue clamping device 200 when the tissue clamping device 200 is delivered to the mitral valve through the left atrial appendage and the left atrium. As shown in FIG. 1, the flexible tube 610 may be located at a front end (i.e., an end close to the tissue clamping device 200) of the delivery tube 600. Correspondingly, the control handle 300 may include a flexible tube control mechanism 620, which may be configured to control the bending of the flexible tube 610. More details about the flexible tube 610 and the flexible tube control mechanism 620 may be found elsewhere in the present disclosure, e.g., FIGs. 7 to 10 and descriptions thereof. By delivering the tissue clamping device 200 to the mitral valve through the left atrial appendage and the left atrium, the operation of mitral valve repair can be more convenient, and the efficiency and the success rate of mitral valve repair can be higher. In addition, when the delivery tube 600 is inflexible or the delivery tube 600 includes a flexible tube 610, the mitral valve repair device 100 may be used for other purposes. For example, the mitral valve repair device 100 may be configured to perform valve repair operations on animals such as pigs.

In some embodiments, as shown in FIGs. 2 to 4, the tissue clamping device 200 may include the inner clamp arm 210, the outer clamp arm 220, a fixing member 230, a supporting part 240, a connecting member 250, and an outer clamping plate 260. The inner clamp arm 210 may include a first inner clamp arm 211 and a second inner clamp arm 213. The outer clamp arm 220 may include a first outer clamp arm 221 and a second outer clamp arm 223. The outer clamping plate 260 may include a first outer clamping plate 261 and a second outer clamping plate 263. One side of the supporting part 240 may be bendably connected to the first outer clamp arm 221 and the first outer clamping plate 261 in sequence. The other side of the supporting part 240 may be bendably connected to the second outer clamp arm 223 and the second outer clamping plate 263 in sequence. The first outer clamp arm 221 and the second outer clamp arm 223 may be bent toward the supporting part 240 and closed relative to each other. The first outer clamp arm 221 and the second outer clamp arm 223 may be bent away from the supporting part 240 and opened relative to each other. The tissue clamping device 200 shown in FIG. 2 is in a state where the first outer clamp arm 221 and the second outer clamp arm 223 are closed relative to each other. The tissue clamping device 200 shown in FIG. 3 is in a state where the first outer clamp arm 221 and the second outer clamp arm 223 are opened relative to each other, and an opening angle they formed (also referred to as an opening angle of the outer clamp arm) is 180°. As used herein, the opening angle refers to an angle between the two outer clamp arms opening to each other. The opening angle of the outer clamp arm may be any angle, such as 10°, 40°, 90°, 120°, 180°, 270°, 350°, 360°. In some embodiments, the outer clamp arm 220, the supporting part 240, and the outer clamping plate 260 may be an integrally formed structure. For example, the outer clamp arm 220, the supporting part 240 and the outer clamping plate 260 may be an integrally formed structure made by cutting and heat-treating a shape memory alloy tube. In some embodiments, as shown in FIG. 2, one end of the supporting part 240 (an upper end shown in the figure) may be connected (e.g., fixedly connected) to the connecting member 250, and one end (a lower end shown in the figure) of the first outer plate 261 and one end (a lower end shown in the figure) of the second outer clamping plate 263 may be respectively connected (e.g., fixedly connected) to the fixing member 230. With this arrangement, when moving relative to the connecting member 250, the fixing member 230 may move relative to the supporting part 240. When the fixing member 230 moves away from the supporting part 240, the first outer clamping plate 261 and the second outer clamping plate 263 may be driven by the fixing member 230, and thus respectively pull the first outer clamp arm 221 and the second outer clamp arm 223 to make them open relatively. In FIGs. 2 to 4, the outer clamp arm control mechanism 400 controls the opening and closing of the outer clamp arm 220 of the tissue clamping device 200 via a driving rod 440. Specifically, one end (a lower end as shown in FIG. 2) of the driving rod 440 may have a threaded structure. The driving rod 440 may be detachably connected to the fixing member 230 by the threaded structure. The outer clamp arm control mechanism 400 may control the movement of the fixing member 230 relative to the connecting member 250 by pushing and pulling the driving rod 440, thereby controlling the opening and closing of the outer clamp arm 220 of the tissue clamping device 200.

In some embodiments, the first inner clamp arm 211 may be disposed on the first outer clamp arm 221, and the second inner clamp arm 213 may be disposed on the second outer clamp arm 223. The first inner clamp arm 211 and the second inner clamp arm 213 may be opened and closed relative to the first outer clamp arm 221 and the second outer clamp arm 223, respectively, which enables the tissue (such as the mitral valve) to be clamped between the first inner clamp arm 211 and the first outer clamp arm 221 and to be clamped between the second inner clamp arm 213 and the second outer clamp arm 223. In some embodiments, the inner clamp arm 210 (i.e., the first inner clamp arm 211 and the second inner clamp arm 213) may be barbed clips. For example, each of movable ends of the inner clamp arm 210 may be disposed with barbs 215. In some embodiments, the inner clamp arm 210 and the outer clamp arm 220 may be connected by bending parts (such as an S-shaped bending structure). The bending parts may have a rebound force, so that the inner clamp arm 210 can closely contact the outer clamp arm 220 in a natural state. In some embodiments, the inner clamp arm control mechanism 500 may control the opening and closing of the inner clamp arm 210 relative to the outer clamp arm 220 by a traction cable (not shown in the figures). For example, the traction cable may be connected to the movable ends of the inner clamp arm 210. When the inner clamp arm control mechanism 500 pulls the traction cable, the inner clamp arm 210 may be opened relative to the outer clamp arm 220 under a pulling force of the traction cable. When the traction cable is relaxed, the inner clamp arm 210 may be closed to the outer clamp arm 220 under the rebound force of the bending parts. In some embodiments, the traction cable may include steel wire, nanowire or glass rope, etc., which is not limited in the present disclosure.

In some embodiments, the delivery tube 600 may be detachably connected to the tissue clamping device 200 by the delivery connection member 700. The delivery connection member 700 may be disposed with through holes, through which the driving rod 440 and the traction cable may pass respectively. In FIGs. 2 and 4, the delivery connection member 700 may include a main body 710, a first connection piece 720, and a second connection piece 730. There may be a rebound force at the connections between the first connection piece 720 and the main body 710 and between the second connection piece 730 and the main body 710, which may make the first connection piece 720 and the second connection piece 730 automatically open in a natural state. Fixed supporting rods 740 may be disposed on the middle of the first connection piece 720 and the second connection piece 730 and perpendicular to the first connection piece 720 and the second connection piece 730, respectively. Suspended ends of the fixed supporting rods 740 may be provided with through holes, through which the driving rod 440 may pass. When the delivery connection member 700 is connected to the connecting member 250 of the tissue clamping device 200, the first connection piece 720 and the second connection piece 730 may be relatively closed and engaged with protrusions of the connecting member 250, respectively. At this time, the driving rod 440 may pass through the through holes of the fixed supporting rods 740 connected to the first connection piece 720 and the second connection piece 730, and restrict the opening of the first connection piece 720 and the second connection piece 730. When the delivery connection member 700 needs to be separated from the tissue clamping device 200, the driving rod 440 may be first disconnected from the tissue clamping device 200 (such as the fixing member 230), and then drawn out until separated from the through holes of the fixed supporting rods 740 connected to the first connection piece 720 and the second connection piece 730. Then, the first connection piece 720 and the second connection piece 730 may be disengaged from the protrusions of the connecting member 250 and automatically opened. In some embodiments, the delivery connection member 700 may be an integrally formed structure made by cutting and heat-treating a shape memory alloy tube.

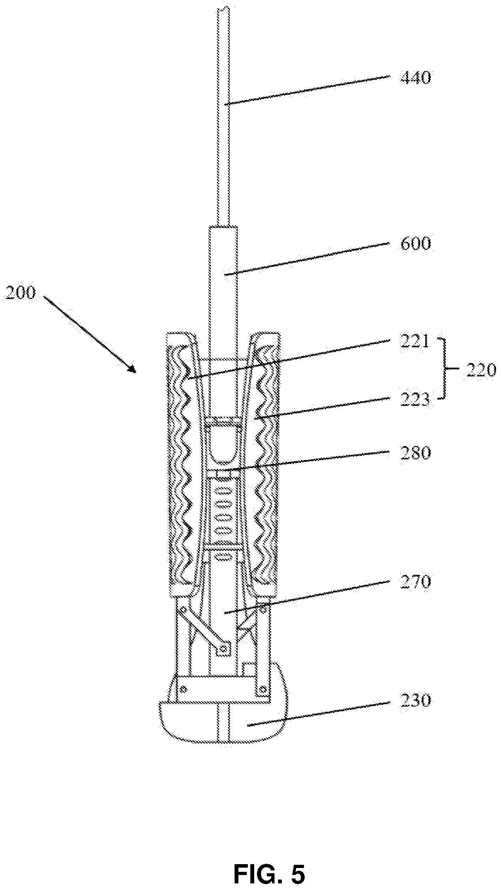

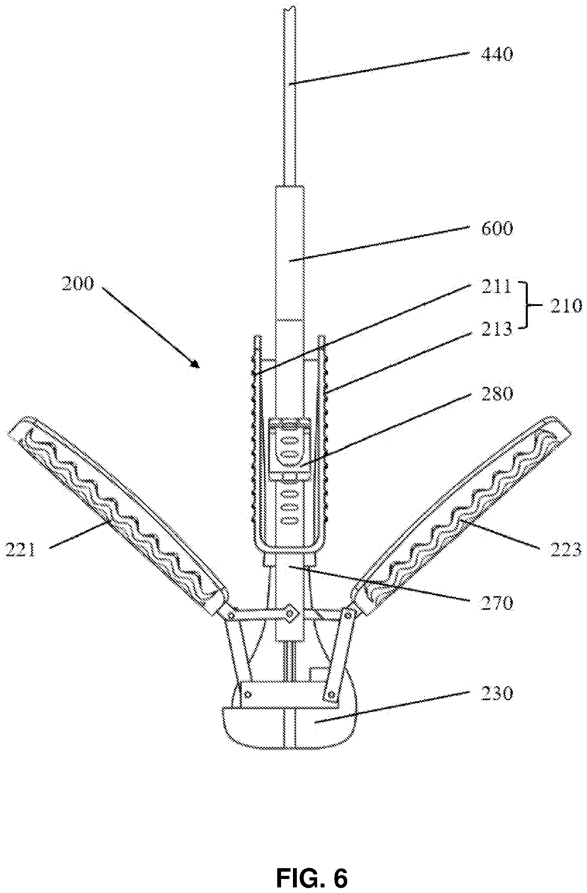

In some embodiments, the tissue clamping device 200 may have other alternative structural forms. For example, FIG. 5 is a structural schematic diagram illustrating a tissue clamping device in a closed state according to another embodiment of the present disclosure. FIG. 6 is a structural schematic diagram illustrating the tissue clamping device in an opened state according to another embodiment of the present disclosure. In FIGs. 5 and 6, a tissue clamping device 200 may include a fixing member 230, an inner clamp arm 210, and an outer clamp arm 220. The inner clamp arm 210 may include a first inner clamp arm 211 and a second inner clamp arm 213. The inner clamp arm 210 may be disposed on the fixing member 230. The first inner clamp arm 211 and the second inner clamp arm 213 may be closed or opened relative to each other. The outer clamp arm 220 may include a first outer clamp arm 221 and a second outer clamp arm 223. The first outer clamp arm 221 and the second outer clamp arm 223 may be both disposed on the fixing member 230. The first outer clamp arm 221 and the second outer clamp arm 223 may be relatively closed or opened, which enables a valve (such as a mitral valve) to be clamped between the first inner clamp arm 211 and the first outer clamp arm 221 and to be clamped between the second inner clamp arm 213 and the second outer clamp arm 223.

In FIGs. 5 and 6, the tissue clamping device 200 may further include a connection pipe 270 and an elastic locking member 280. The delivery tube 600 may be detachably connected (such as in a screw connection) with the connection pipe 270. The driving rod 440 may be directly or indirectly connected with the fixing member 230. The outer clamp arm 220 may be rotatably connected to the fixing member 230 and the connection pipe 270 by a connection rod structure. The outer clamp arm control mechanism 400 may control the movement of the fixing member 230 relative to the connection pipe 270 by pushing and pulling the driving rod 440, and then control the opening and closing of the outer clamp arm 220 of the tissue clamping device 200. When the outer clamp arm 220 is opened to a certain angle (such as 10°, 90°, 150°, 180°, etc.) or closed, the elastic locking member 280 disposed on the fixing member 230 may cooperate with bayonet(s) on the connection pipe 270, thereby forming a certain resistance on the opening and closing of the outer clamp arm 220. In some embodiments, the inner clamp arm 210 may be an integrally formed U-shaped structure and have a rebound force that forces it to expand outwardly. The inner clamp arm control mechanism 500 may control the opening and closing of the inner clamp arm 210 relative to the outer clamp arm 220 by a traction cable (not shown). For example, the traction cable may be connected to the movable ends of the inner clamp arm 210. When the inner clamp arm control mechanism 500 pulls the traction cable, the inner clamp arm 210 may be opened relative to the outer clamp arm 220 under the pulling force of the traction cable (that is, the first inner clamp arm 211 and the second inner clamp arm 213 are closed to each other). When the traction cable is relaxed, the inner clamp arm 210 may expand outwardly under the rebound force to be closed with the outer clamp arm 220. Specifically, the first inner clamp arm 211 and the first outer clamp arm 221 may be controlled separately to open or close relative to the first outer clamp arm 221 and the second outer clamp arm 223, respectively.

Hereinafter, the control handle 300 may be described by taking the tissue clamping device 200 shown in FIGs. 2 to 4 as an example. It should be noted that the control handle 300 described in the following description is merely for illustrative purposes only, and the present disclosure is not intended to be limiting. Part or whole of the control handle 300 may be applied not only to the tissue clamping device 200 shown in FIGs. 2 to 4, but also to other types of tissue clamping devices (for example, the tissue clamping device 200 shown in FIGs. 5 and 6).

The control handle 300 includes the outer clamp arm control mechanism 400 and the inner clamp arm control mechanism 500. The outer clamp arm control mechanism 400 is be configured to control the movement of the outer clamp arm 220 of the tissue clamping device 200. The inner clamp arm control mechanism 500 is configured to control the movement of the inner clamp arm 210 of the tissue clamping device 200.

In some embodiments, when the delivery tube 600 includes the flexible tube 610, the control handle 300 may include a flexible tube control mechanism 620 that may be configured to control the bending of the flexible tube 610. In some embodiments, when the delivery tube 600 does not include the flexible tube 610 (for example, when the delivery tube 600 is an inflexible structure), the control handle 300 may include no flexible tube control mechanism 620.

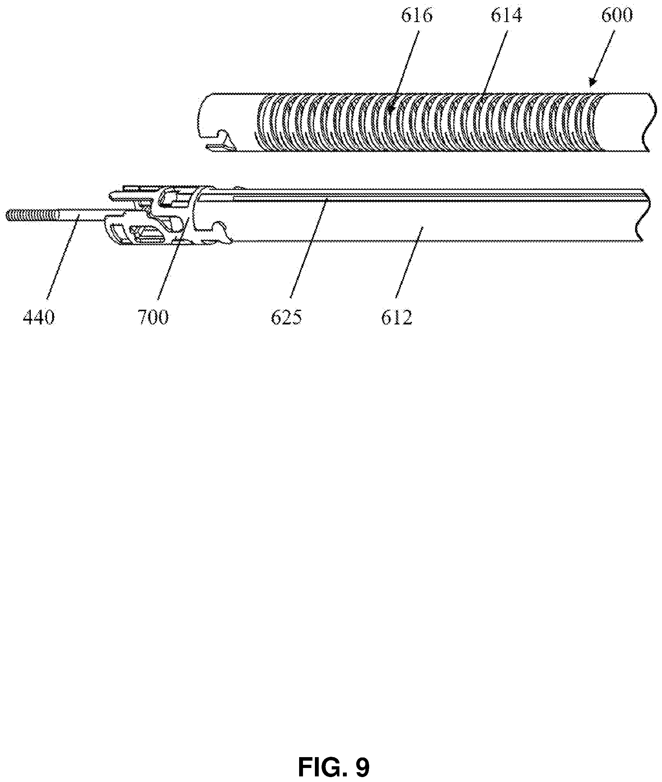

FIG. 7 is a structural schematic diagram illustrating a flexible tube from a front view according to some embodiments of the present disclosure. FIG. 8 is a structural schematic diagram illustrating the flexible tube from a rear view according to some embodiments of the present disclosure. FIG. 9 is a structural schematic diagram illustrating different parts of the flexible tube according to some embodiments of the present disclosure. As shown in FIGs. 7 to 9, the flexible tube 610 may be connected to the front end (i.e., an end close to the tissue clamping device 200) of the delivery tube 600. For example, the flexible tube 610 may be connected to (such as laser welding) or integrally formed with the body of the delivery tube 600. The front end of the flexible tube 610 may be connected to the delivery connection member 700. In some embodiments, the flexible tube 610 may include an inner core 612 and an outer pipe 614. The outer pipe 614 may be sleeved outside the inner core 612. One end of the delivery connection member 700 may be sleeved outside the inner core 612 and engaged with the outer pipe 614. In some embodiments, one or more through holes may be disposed inside the inner core 612 to allow the driving rod 440 and the traction cable that controls the opening and closing of the inner clamp arm 210 to pass through.

In FIGs. 7 to 9, the flexible tube 610 may be disposed with a plurality of notches 616 along a length direction of the flexible tube 610. Specifically, the outer pipe 614 of the flexible tube 610 may be disposed with the plurality of notches 616. By the plurality of notches 616, the flexible tube 610 may be easily bent, and bent in a specific direction. In this embodiment, the plurality of notches 616 may be disposed on one side of the flexible tube 610, so that the flexible tube 610 can be bent toward an opening direction of the notches 616. In some embodiments, the plurality of notches 616 may be spaced on different sides of the flexible tube 610, so that the flexible tube 610 can be bent toward a plurality of directions. In some embodiments, the outer pipe 614 of the flexible tube 610 may be made of stainless steel (such as type 316) or an elastic metal (such as nickel-titanium alloy). For example, the outer pipe 614 may be cut from a stainless steel tube or an elastic metal tube. The inner core 612 of the flexible tube 610 may be made of an elastic material (such as nylon, silicone, heat shrinkable polyether block polyamide (Pebax or VES-TAMID), polytetrafluoroethylene (PTFE)). In some embodiments, the flexible tube 610 may have elasticity. When there is no external force, the flexible tube 610 may maintain a cylindrical shape. Further, a polymer material layer (such as a heat-shrinkable polyether block polyamide (Pebax)) may also be disposed on an outer surface of the flexible tube 610, which can effectively prevent the flexible tube 610 from contacting blood. In some embodiments, the plurality of notches 616 may be disposed on one side of the flexible tube 610, and a side of the inner core 612 facing the opening direction of the notches 616 may be disposed with a groove, and a traction rope 625 may be disposed in the groove. Specifically, the front end of the traction rope 625 may be fixedly connected (such as welding connection, glue connection) to the inner core 612 and/or the outer pipe 614. The flexible tube control mechanism 620 may control the bending of the flexible tube 610 by pulling the traction rope 625.

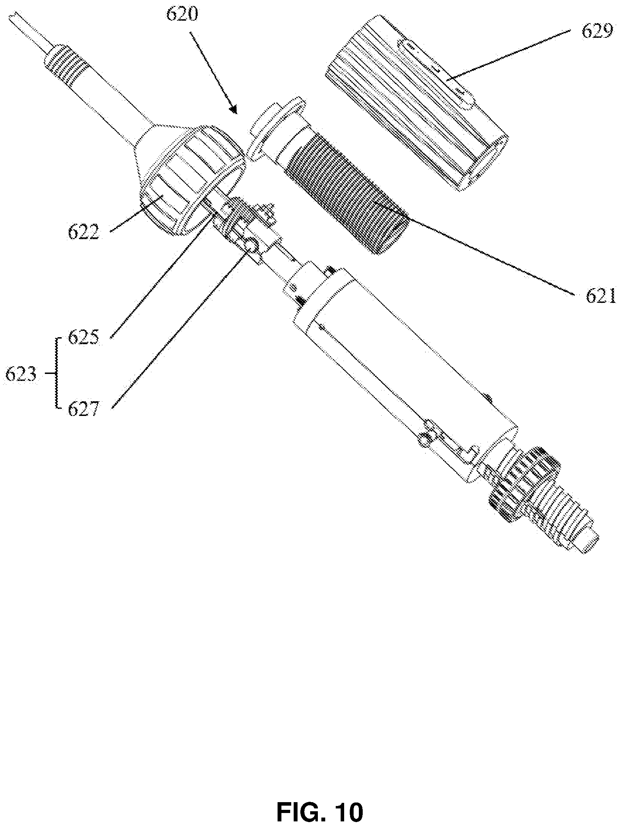

FIG. 10 is a structural schematic diagram illustrating different parts of a flexible tube control mechanism according to some embodiments of the present disclosure. In some embodiments, as shown in FIG. 10, the flexible tube control mechanism 620 may include a screw 621, a rotating part 622, and a traction part 623. The screw 621 and the traction part 623 may be threadedly connected. The rotating part 622 may drive the screw 621 to rotate, thereby driving the traction part 623 to move. The movement of the traction part 623 may control the flexible tube 610 to bend. In some embodiments, the traction part 623 may include the traction rope 625 and a threaded traction block 627. One end (such as a rear end) of the traction rope 625 may be connected to the threaded traction block 627, and the other end (such as a front end) of the traction rope 625 may be fixedly connected to the front end of the flexible tube 610. The front end herein refers to the left end of the traction rope 625 in FIG. 9. An internal thread may be disposed in the screw 621, and the threaded traction block 627 may be movably disposed in the screw 621 and cooperate with the internal thread of the screw 621. When the rotating part 622 drives the screw 621 to rotate, the screw 621 may drive the threaded traction block 627 to move, along the length direction, in the screw 621, so as to realize the traction or loosening of the traction rope 625, thereby controlling the bending of the flexible tube 610. Specifically, the traction of the flexible tube 610 by the traction rope 625 may cause the plurality of notches 616 on the flexible tube 610 to be closed with each other, so that the flexible tube 610 bends. When the threaded traction block 627 stops moving, the flexible tube 610 may maintain a bending state. When the threaded traction block 627 loosens the traction rope 625, the flexible tube 610 may reduce a bending degree under its own elastic force until returning to a natural state (such as maintaining a cylindrical shape).

In some embodiments, as shown in FIG. 10, the flexible tube control mechanism 620 may include a bending indication device 629. The bending indication device 629 may be configured to indicate a bending degree of the flexible tube 610. In some embodiments, the bending indication device 629 may include an indication block. The indication block may be engaged with an external thread of the screw 621 and move with the rotation of the screw 621. A position the indication block moves to may reflect the bending degree of the flexible tube 610. In some embodiments, a position the indication block moves to may correspond to a bending angle of the flexible tube 610 one to one. The corresponding relationship between the position the indication block moves to and the bending angle of the flexible tube 610 may be determined by experiments. In some embodiments, the bending indication device 629 may further include an indication mark. The indication mark may be disposed on a housing (such as a transparent housing covering the outside of the indication block) to intuitively reflect the bending degree (such as a bending angle) of the flexible tube 610 corresponding to a position the indication block moves to.

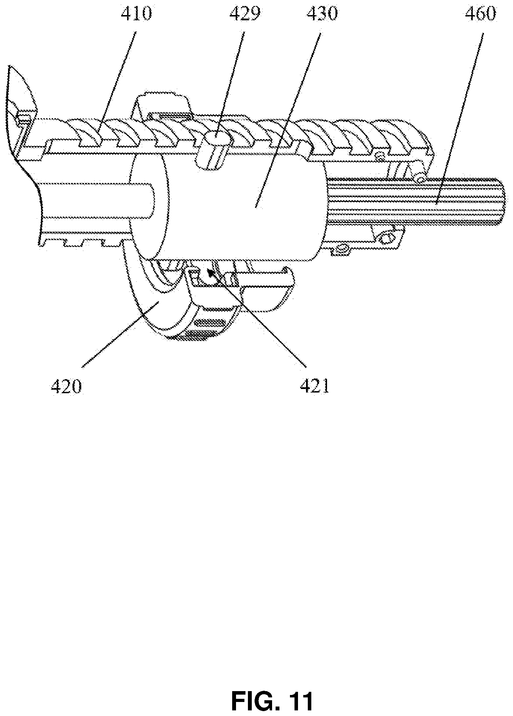

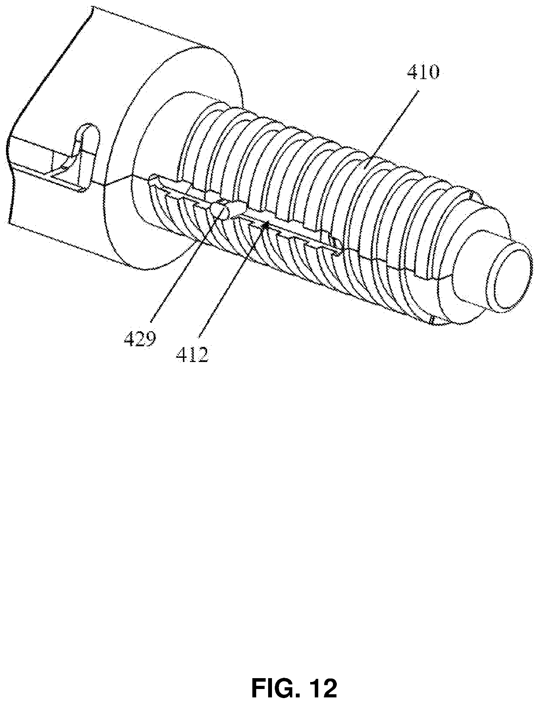

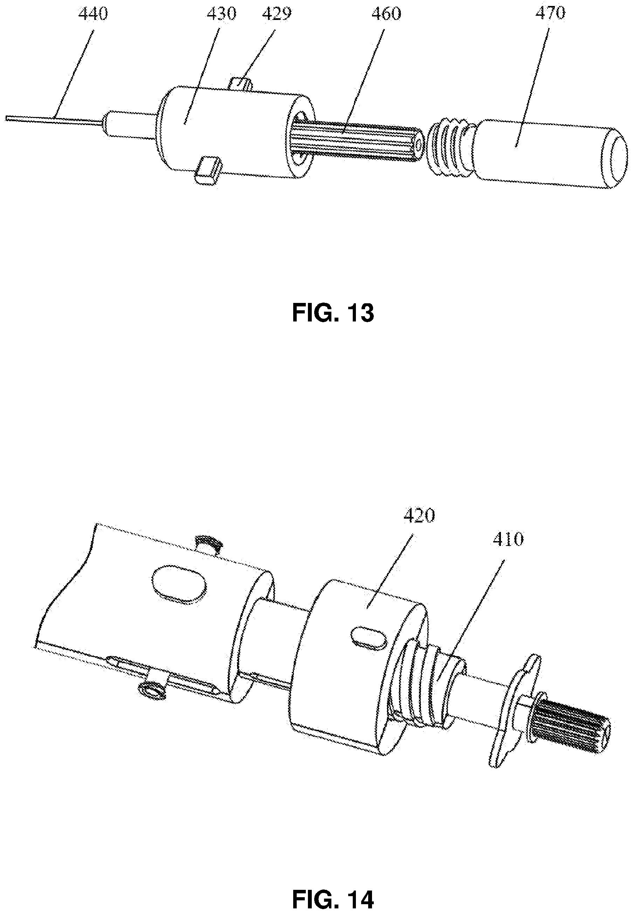

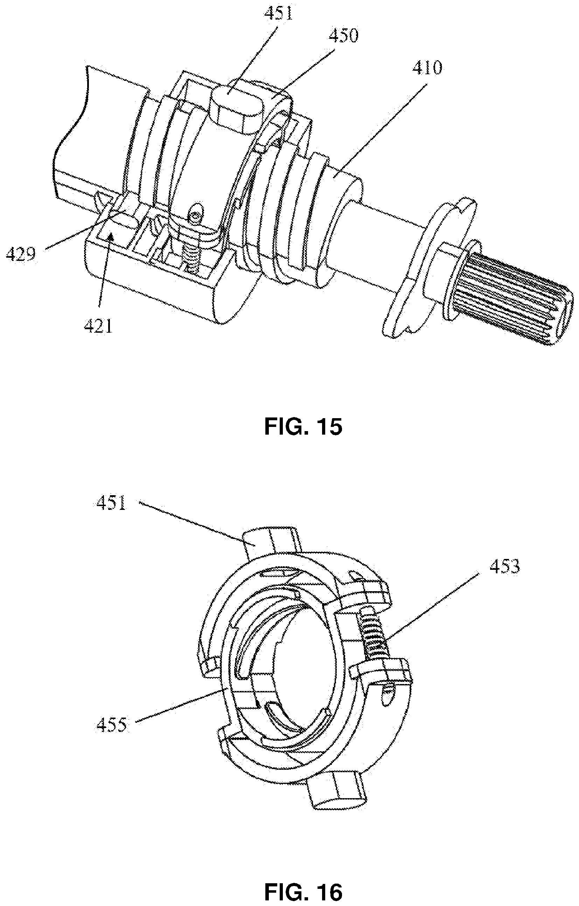

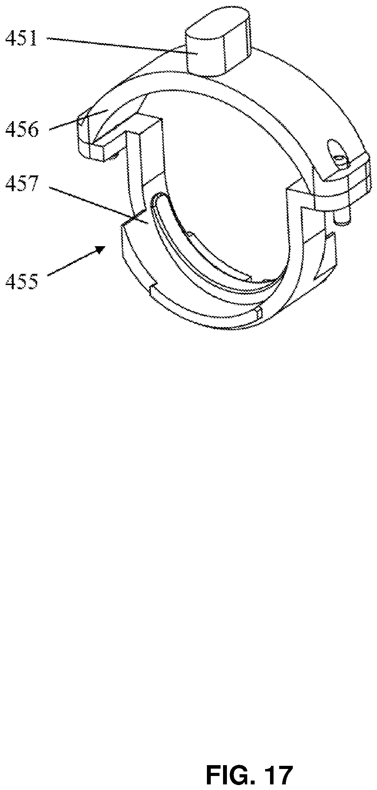

FIG. 11 is a partial sectional diagram illustrating an outer clamp arm control mechanism according to some embodiments of the present disclosure. FIG. 12 is a structural schematic diagram illustrating a sleeve according to some embodiments of the present disclosure. FIG. 13 is a structural schematic diagram illustrating a sliding part and a protective cover according to some embodiments of the present disclosure. FIG. 14 is a structural schematic diagram illustrating an outer clamp arm control mechanism according to another embodiment of the present disclosure. FIG. 15 is a schematic diagram illustrating an internal structure of the outer clamp arm control mechanism according to another embodiment of the present disclosure. FIG. 16 is a structural schematic diagram illustrating a threaded engagement mechanism according to another embodiment of the present disclosure. FIG. 17 is a structural schematic diagram illustrating an engagement member of the threaded engagement mechanism according to another embodiment of the present disclosure. As shown in FIGs. 11 to 16, the outer clamp arm control mechanism 400 includes a sleeve 410, a first control part 420, and a sliding part 430. The sliding part 430 is disposed in the sleeve 410. The first control part 420 rotates to drive the sliding part 430 to move in the sleeve 410 and along a length direction of the sleeve 410, so as to control the opening and closing of the outer clamp arm 220. If one end of the control handle 300 close to the tissue clamping device 200 is defined as a front end, and the other end of the control handle 300 is defined as a rear end, when the sliding part 430 moves toward the front end in the sleeve 410, the opening of the outer clamp arm 220 may be controlled to open (such as the first outer clamp arm 221 and the second outer clamp arm 223 are opened relative to each other). When the sliding part 430 moves toward the rear end, the outer clamp arm 220 may be controlled to close (such as the first outer clamp arm 221 and the second outer clamp arm 223 are closed relative to each other).

In some embodiments, the sleeve 410 may include one or more interlayers. The sliding part 430 may be disposed in the interlayers of the sleeve 410. The first control part 420 may drive the sliding part 430 to move in the sleeve 410 and along the length direction of the sleeve 410. For example, the sleeve 410 may have a hollow cylindrical shape, which may be formed by connecting two semi-cylindrical housings. The sliding part 430 may have a cylindrical shape and may be clamped between the two semi-cylindrical housings of the sleeve 410.

In some embodiments, referring to FIGs. 11 and 12, an external thread may be disposed on an outer circumferential surface of the sleeve 410. An internal thread may be disposed on an inner circumferential surface of the first control part 420. The sleeve 410 and the first control part 420 may be connected by the threads (i.e., the outer thread and the inner thread). One or more first sliding grooves 412 are opened on the sleeve 410 along the length direction thereof. The sliding part 430 may be connected to the first control part 420 by passing through the first sliding groove(s) 412. Specifically, the sliding part 430 may include one or more protruding connection block(s) 429. The inner circumferential surface of the first control part 420 may include one or more connection grooves 421. The connection block(s) 429 may protrude from the first sliding groove(s) 412 and be clamped with the connection groove(s) 421, respectively, so that the first control part 420 may rotate to drive the sliding part 430 to move along the first sliding groove(s) 412. In some embodiments, the sleeve 410 may include two first sliding grooves 412, which are respectively disposed on two sides of the sleeve 410. The sliding part 430 may include two protruding connection blocks 429 corresponding to the two first sliding grooves 412, respectively, thereby ensuring the stability of the movement of the sliding part 430 driven by the first control part 420. In some alternative embodiments, a count of the first sliding grooves 412 may be other numbers, such as one, three, five.

In some embodiments, the first control part 420 may have a circular outer contour, and a rubber layer may be disposed on a surface of the outer contour. When an operator controls the outer clamp arm 220 by rotating the first control part 420, the rubber layer can increase the friction between the first control part 420 and the palm or fingers, so that the operator can precisely control the first control part 420. In some other embodiments, a layer made of hard materials such as plastic, metal may be disposed on the surface of the outer contour of the first control part 420 without the rubber layer, and anti-slip patterns may be added on the layer to increase the friction.

As shown in FIGs. 11 to 13, the outer clamp arm control mechanism 400 includes a driving rod 440, a fixing block 460, and a protective cover 470. The sliding part 430 controls the opening and closing of the outer clamp arm 220 by the driving rod 440, and a rear end (e.g., a right end in FIG. 13) of the driving rod 440 is fixedly connected to the fixing block 460. Specifically, the fixing block 460 may be cylindrical, and a cross-sectional diameter thereof may be larger than that of the driving rod 440. The driving rod 440 may be inserted into and fixedly connected to the fixing block 460 in a manner such as glue connection, welding connection, or interference connection. The protective cover 470 is detachably connected to the sliding part 430 by a thread. When connected to the sliding part 430, the protective cover 470 restricts the relative movement of the fixing block 460 and the sliding part 430. In some embodiments, the driving rod 440 may be made of a memory alloy (such as a nickel-titanium alloy), so that the driving rod 440 can have relatively good tensile and compressive properties and relatively good bending performance. Thus, although the delivery tube 600 is bent, the outer clamp arm control mechanism 400 may also effectively control the opening and closing of the outer clamp arm 220 by the driving rod 440.

In some embodiments, after the clamping of the tissue clamping device 200 is completed, the driving rod 440 needs to be separated from the tissue clamping device 200, and part or whole of the driving rod 440 may be drawn out from the control handle 300. As shown in FIGs. 11 to 13, the protective cover 470 may be detachably connected to the sliding part 430 by the thread. When the driving rod 440 needs to be separated from the tissue clamping device 200, the operator may rotate the protective cover 470 to separate the driving rod 440 from the sliding part 430, rotate the fixing block 460 (that is, rotate the driving rod 440) to separate the driving rod 440 from the tissue clamping device 200, and pull the fixing block 460 to draw out the driving rod 440 from the control handle 300.

In some embodiments, as shown in FIGs. 14 to 17, the first control part 420 may include a thread engagement mechanism 450. The thread engagement mechanism 450 may include manipulation buttons 451, a first elastic member 453 and a pair of engagement members 455. The engagement members 455 may be symmetrically disposed (e.g., central symmetrically) and configured to be engaged with the external thread of the sleeve 410 by an elastic force of the first elastic member 453. The manipulation buttons 451 may be disposed on outer sides of the engagement members 455, and configured to control the engagement members 455 to be separated from the external thread of the sleeve 410 by overcoming the elastic force of the first elastic member 453. As shown in FIGs. 14 to 17, each of the engagement members 455 may include a pressing part 456 and an engagement part 457. The pressing part 456 and the engagement part 457 may be fixedly connected or in an integrally formed structure. An inner circumferential surface of the engagement part 457 may be disposed with a tooth structure that matches with the external thread of the sleeve 410. A pair of engagement members 455 may be disposed oppositely and looped on the outer circumferential surface of the sleeve 410. Under the action of the first elastic member 453, the engagement part 457 of each of the two engagement members 455 may be engaged with the external thread of the sleeve 410. In this embodiment, the first elastic member 453 may include two springs, which are respectively disposed at both sides of the pair of engagement members 455.

In the actual operation, the operator may press the two manipulation buttons 451 that may drive the pair of engagement members 455 to relatively move and thereby compress the first elastic member 453, such that the tooth structure inside the engagement members 455 is separated from the external thread of the sleeve 410. Therefore, the first control part 420 and the sleeve 410 may slide relatively in the length direction of the sleeve 410. At this time, the operator may directly drag the first control part 420 to slide on the sleeve 410, thereby achieving quick opening and closing of the outer clamp arm 220. By controlling the quick opening and closing of the outer clamp arm 220, the mitral valve repair device or control handle thereof can be operated more flexibly during surgery (such as mitral valve repair surgery) and suitable for different surgical conditions. When the operator releases the control button, the engagement members 455 may be again engaged with the external thread of the sleeve 410 under the elastic force of the first elastic member 453, and the operator may rotate the first control part 420 as needed to adjust the opening angle of the outer clamp arm 220 or perform the next operation. By disposing two opposite manipulation buttons 451, it can be convenient for the operator to control the quick opening and closing of the outer clamp arm 220, and effective to prevent the operator from misoperation due to accidental touch.

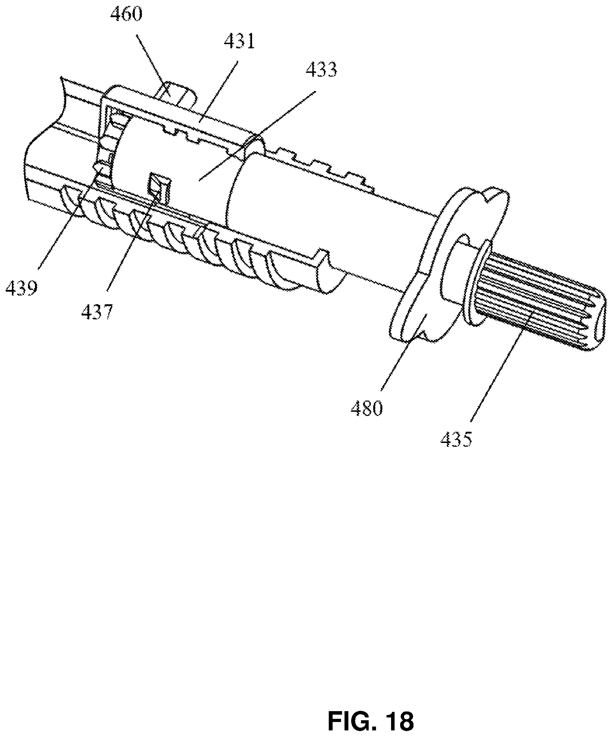

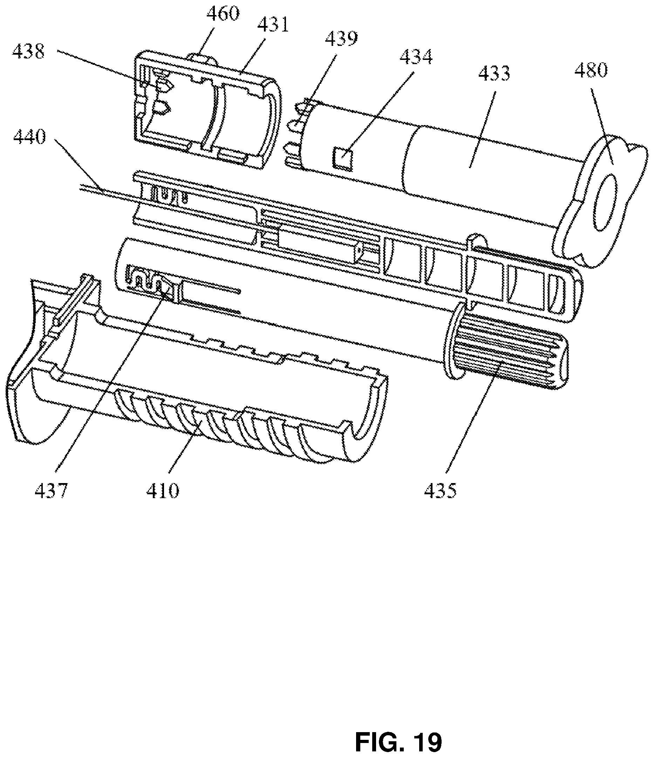

FIG. 18 is a partial sectional diagram illustrating a sliding part according to another embodiment of the present disclosure. FIG. 19 is a structural schematic diagram illustrating different parts of the sliding part according to another embodiment of the present disclosure. As shown in FIGs. 18 and 19, the sliding part 430 may include an outer cover 431, a middle tube 433, and an inner pipe 435. The middle tube 433 may be sleeved outside the inner pipe 435. One or more opening 434 may be disposed on the middle tube 433. One or more fixture blocks 437 that can extend outward may be disposed on the inner pipe 435. The fixture block(s) 437 may be engaged with the outer cover 431 by passing through the opening(s) 434, so as to restrict the relative movement of the middle tube 433, the inner pipe 435, and the outer cover 431 in the length direction of the sleeve 410. The driving rod 440 may be fixedly connected to the inner pipe 435. For example, one end of the driving rod 440 may be fixed in the inner pipe 435 by means of locking connection, glue connection, interference connection, etc. In some embodiments, the fixture block(s) 437 on the inner pipe 435 may have a wedge-shaped outer contour. Groove(s) corresponding to the fixture block(s) 437 may be disposed on the inner wall of the outer cover 431. The fixture block(s) 437 may pass through the opening(s) 434 of the middle tube 433 to insert into the groove(s), so as to restrict the relative movement of the outer cover 431, the middle tube 433, and the inner pipe 435 in the length direction of the sleeve 410.

In some embodiments, the fixture block(s) 437 may be pressed into the middle tube 433. Specifically, the fixture block(s) 437 may be connected to the inner pipe 435 by a bendable structure. For example, one end of each of the fixture block(s) 437 may be connected to an outer wall of the inner pipe 435 by an elastic rod, and the other end thereof may be connected to the outer wall of the inner pipe 435 by the bendable structure having an "S" shape. As another example, one end of each of the fixture block(s) 437 may be suspended, and the other end thereof may be connected to the outer wall of the inner pipe 435 by the bendable structure. When the fixture block(s) 437 are subjected to a squeezing force, the bendable structure may be bent inwardly so that the fixture block(s) 437 are pressed into the middle tube 433 and separated from the opening(s) 434. In some embodiments, there may be one or more fixture blocks 437. In this embodiment, there may be two fixture blocks 437, which are symmetrically disposed, and two openings 434 corresponding to the fixture blocks 437 may be disposed on the middle tube 433, respectively. As another example, there may be 3, 4, 6, etc. fixture blocks 437, which may be disposed annularly along the outside of the inner pipe 435 or along the length direction of the sleeve 410.

As shown in FIGs. 18 and 19, a size (such as a width) of each of the fixture block(s) 437 may be matched with that of each of the opening(s) 434, so that the relative rotation between the inner pipe 435 and the middle tube 433 can be restricted by snapping the fixture block(s) 437 into the opening(s) 434, respectively. In some embodiments, one or more locking teeth 439 may be disposed at a front end (e.g., a left end in FIG. 19) of the middle tube 433. One or more locking grooves 438 corresponding to the locking teeth 439 may be disposed at the outer cover 431 (such as the front end (e.g., the left end in FIG. 19) of an inner circumferential surface of the outer cover 431). The one or more locking teeth 439 may be engaged with the one or more locking grooves 438 to restrict the relative rotation of the middle tube 433 and the outer cover 431. In some embodiments, there may be one or more locking teeth 439 and the locking grooves 438. In some embodiments, there may be a plurality of (e.g., two or more, three or more) locking teeth 439 and locking grooves 438, which are disposed at equal intervals along a circumferential direction of the outer cover 431 and the middle tube 43 to facilitate matching during assembly. In some embodiments, the positions of the above-mentioned locking grooves 438 and the locking teeth 439 may be reversed, which can achieve the same technical effect. For example, the one or more locking grooves may be disposed on an outer circumferential surface of the middle tube 433, and the one or more locking teeth 439 may be disposed on the inner circumferential surface of the outer cover 431.

In some embodiments, a rear end (e.g., a right end in FIG. 19) of the middle tube 433 may be disposed with a boss 480, and the rear end (e.g., a right end in FIG. 19) of the inner pipe 435 may extend from the middle tube 433. By pulling the boss 480 and/or pushing the rear end of the inner pipe 435, the one or more locking teeth 439 may be separated from the locking groove(s) 438, and the fixture block(s) 437 may be pressed into the middle tube 433. By disposing the boss 480, the operator can conveniently separate the middle tube 433 from the outer cover 431, and can easily release the restriction on the relative rotation between the inner pipe 435 and the middle tube 433. After the locking teeth 439 are separated from the locking groove(s) 438 and the fixture block(s) 437 are pressed into the middle tube 433, the operator may rotate the inner pipe 435 (that is, rotate the driving rod 440) to separate the driving rod 440 from the tissue clamping device 200. The operator may then pull the inner pipe 435 (or the middle tube and the inner pipe) to draw out the driving rod 440 from the control handle 300 (e.g., draw out part or whole of the driving rod 440).

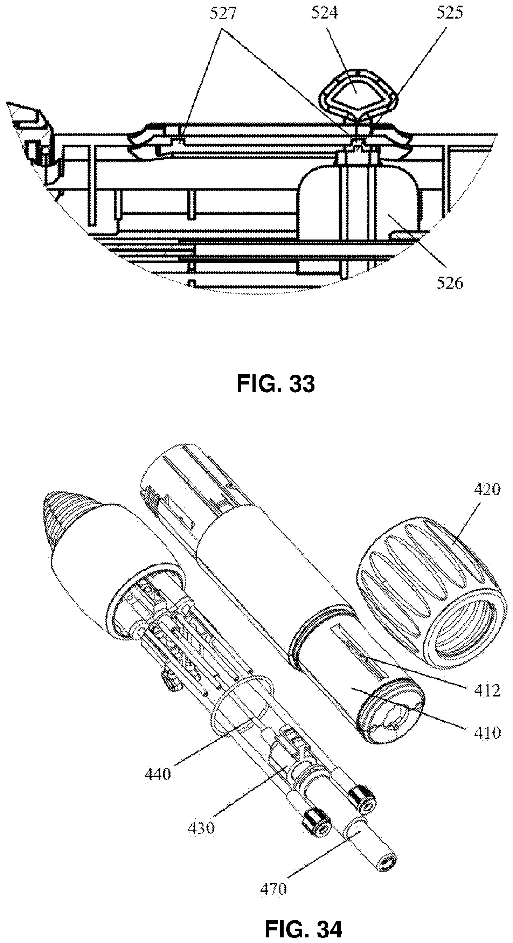

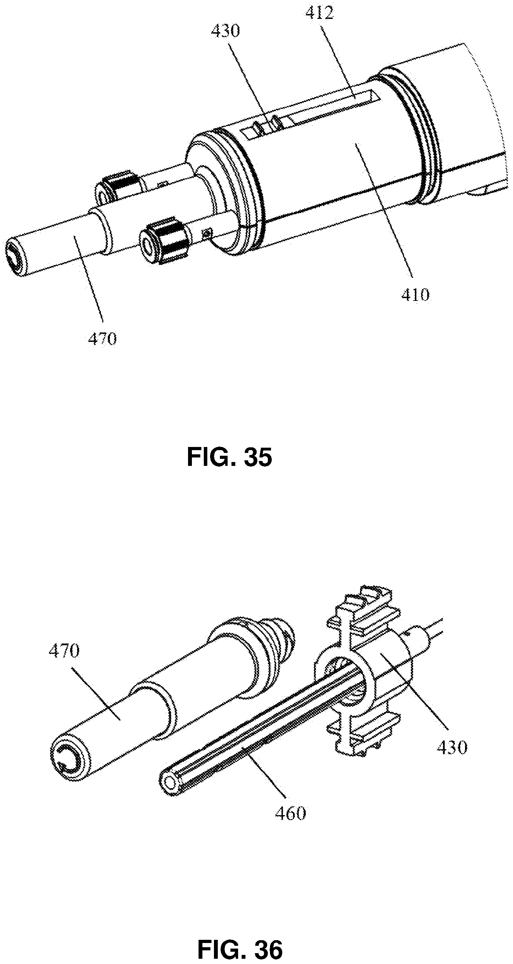

FIG. 34 is a schematic structural diagram illustrating an outer clamp arm control mechanism according to another embodiment of the present disclosure. FIG. 35 is a partial structural diagram illustrating an outer clamp arm control mechanism according to another embodiment of the present disclosure. FIG. 36 is a structural schematic diagram illustrating a sliding part according to another embodiment of the present disclosure. In some embodiments, as shown in FIGs. 34 and 36, an outer clamp arm control mechanism 400 may include a sleeve 410, a first control part 420, and a sliding part 430. The sliding part 430 may be disposed in the sleeve 410. The first control part 420 may rotate to drive the sliding part 430 to move in the sleeve 410 and along a length direction of the sleeve 410, so as to control the opening and closing of the outer clamp arm 220. If one end of the control handle 300 close to the tissue clamping device 200 is designated as the front end, and the other end of the opposite control handle 300 is designated as the rear end, when the sliding part 430 moves toward the front end in the sleeve 410, the opening of the outer clamp arm 220 may be controlled to open (such as the first outer clamp arm 221 and the second outer clamp arm 223 are opened relative to each other). When the sliding part 430 moves toward the rear end, the outer clamp arm 220 may be controlled to close (such as the first outer clamp arm 221 and the second outer clamp arm 223 are closed relative to each other).

In some embodiments, one or more internal threads may be disposed on the inner circumferential surface of the first control part 420, one or more external threads may be disposed on the sliding part 430, and one or more first sliding grooves 412 may be disposed on the sleeve 410 along the length direction. The sliding part 430 may pass through the first sliding groove(s) 412 and the external thread(s) on the sliding part 430 may cooperate with the internal thread(s) of the first control part 420, so that the first control part 420 may rotate to drive the sliding part 430 move along the first sliding groove(s) 412. In some embodiments, two first sliding grooves 412 may be disposed on the sleeve 410, which are respectively located on opposite sides of the sleeve 410, and two external threads are correspondingly disposed on the sliding part 430. The two external threads may respectively pass through the two first sliding grooves 412 to protrude outward and cooperate with the internal threads of the first control part 420. By disposing two first sliding grooves 412 and two external threads, the stability of the movement of the sliding part 430 driven by the first control part 420 can be ensured. In some alternative embodiments, a count of the first sliding grooves 412 may be one, three, five, etc.

In some embodiments, as shown in FIGs. 34 and 36, the outer clamp arm control mechanism 400 may include a driving rod 440, a fixing block 460, and a protective cover 470. The sliding part 430 may be connected to the driving rod 440 and control the opening and closing of the outer clamp arm 220 by the driving rod 440, and the rear end of the driving rod 440 may be fixedly connected to the fixing block 460. Specifically, the fixing block 460 may be cylindrical, and a cross-sectional diameter thereof may be larger than a cross-sectional diameter of the driving rod 440. The driving rod 440 may be inserted into the fixing block 460 and fixedly connected to the fixing block 460 in a manner such as glue connection, welding connection, or interference connection. The protective cover 470 may be detachably connected to the sliding part 430 by a thread. When the protective cover 470 is connected to the sliding part 430, the protective cover 470 may restrict the relative movement of the fixing block 460 and the sliding part 430. In some embodiments, the driving rod 440 may be made of a memory alloy (such as a nickel-titanium alloy), so that the driving rod 440 can have relatively good tensile and compressive properties and relatively good bending performance. Furthermore, when the delivery tube 600 is bent, the outer clamp arm control mechanism 400 may effectively control the opening and closing of the outer clamp arm 220 by the driving rod 440.

In some embodiments, the sleeve 410 may be connected to (or integrally formed with) one end of a housing 510, and central axes of the housing 510 and the sleeve 410 may coincide, making the control handle 300 more compact and easier to handle. For example, the housing 510 may be disposed at an end of the sleeve 410 near the tissue clamping device 200. In some alternative embodiments, the housing 510 may be disposed at an end of the sleeve 410 away from the tissue clamping device 200.

In some embodiments, the first control part 420 may have a circular outer contour, and a rubber layer may be disposed on a surface of the outer contour. When the operator controls the outer clamp arm 220 by rotating the first control part 420, the rubber layer can increase the friction between the first control part 420 and the palm or fingers, so that the operator can precisely control the first control part 420. In some embodiments, a layer made of hard materials such as plastic, metal may be disposed on the surface of the outer contour of the first control part 420 without the rubber layer, and anti-slip patterns may be added on the layer to increase the friction.

In some embodiments, after the clamping of the tissue clamping device 200 is completed, the driving rod 440 needs to be separated from the tissue clamping device 200 and part or whole of the driving rod 440 may be drawn out from the control handle 300. As shown in FIG. 36, the protective cover 470 may be detachably connected to the sliding part 430 by the thread. When the driving rod 440 needs to be separated from the tissue clamping device 200, the operator may rotate the protective cover 470 to separate the driving rod 440 from the sliding part 430, rotate the fixing block 460 (that is, rotate the driving rod 440) to separate the driving rod 440 from the tissue clamping device 200, and pull the fixing block 460 to draw out the driving rod 440 (e.g., part or whole) from the control handle 300.

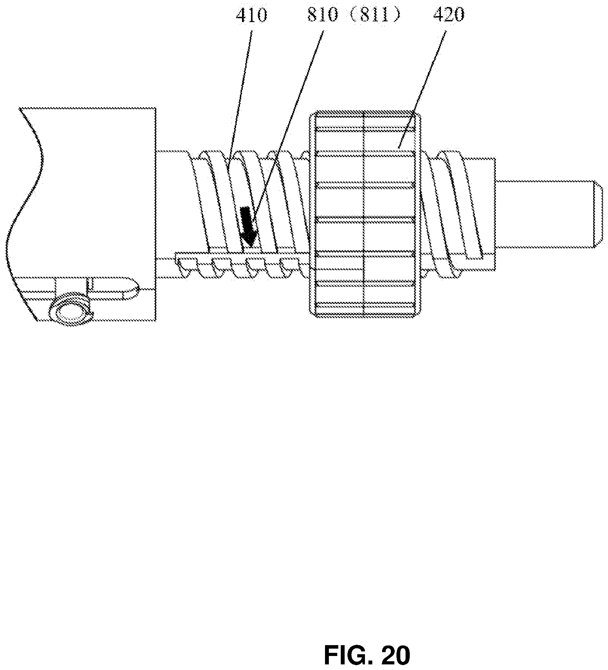

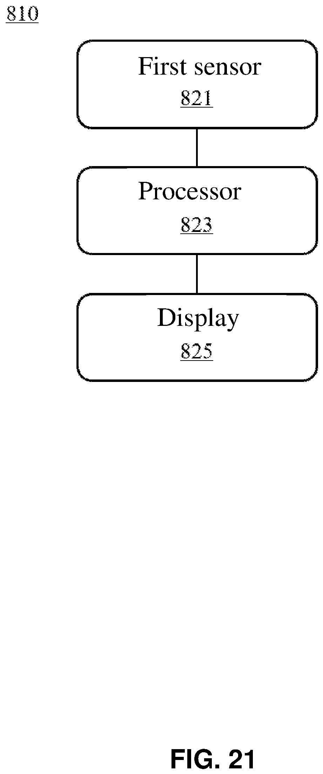

In some embodiments, the outer clamp arm control mechanism 400 may include a first indicating device 810. The first indicating device 810 may be configured to indicate an opening angle of the outer clamp arm 220 according to the position of the first control part 420 and/or the position of the sliding part 430. FIG. 20 is a structural schematic diagram illustrating an outer clamp arm control mechanism which can indicate an opening angle of the outer clamp arm according to some embodiments of the present disclosure. FIG. 21 is a schematic diagram illustrating a first indicating device according to some embodiments of the present disclosure. By disposing the first indicating device 810, the operator can more conveniently and intuitively control the opening angle of the outer clamp arm 220 during surgery or experiments.

In some embodiments, as shown in FIG. 20, the first indicating device 810 may include one or more angle marks 811 disposed on the sleeve 410. As shown in FIG. 20, the angle mark(s) 811 may include mark(s) (such as arrow(s)) engraved or printed on the sleeve 410. The first control part 420 may rotate to align with the angle mark(s) 811 (for example, the front end of the first control part 420 is at the position indicated by the arrow), indicating that the outer clamp arm 220 is opened to an angle the angle mark(s) 811 represent. In some embodiments, the angle mark(s) 811 may include other forms. For example, the angle mark(s) 811 may include a scale mark, an angle value, an indicator arrow in different colors, etc. As another example, the angle mark(s) 811 may be a paper, a string, etc., that is fixedly connected (e.g., glue connection) to the surface of the sleeve 410 but does not affect the rotation of the first control part 420. In some embodiments, there may be a plurality of angle marks 811, which may be sequentially disposed along the length direction or a spiral direction of the sleeve 410. Each of the plurality of angle marks 811 may correspond to a specific angle (such as 90°, 120°, 180°, etc.) at which the outer clamp arm 220 is opened (i.e., the opening angle of the outer clamp arm 220). In some embodiments, the correspondence between each of the angle marks 811 and the specific angle at which the outer clamp arm 220 is opened may be obtained by experiments. In some embodiments, an installation position of each component may be adjusted according to the specific angle corresponding to each of the angle marks 811 when the outer clamp arm control mechanism 400 is to be installed, so that when the installation is completed, each of the angle marks 811 can correspond to a corresponding specific angle.

In some embodiments, as shown in FIG. 20, the first indicating device 810 may include a processor 823, a display 825, and a first sensor 821. The first sensor 821 may be configured to detect a position of the first control part 420 and/or a position of the sliding part 430. The processor 823 may be configured to determine the opening angle of the outer clamp arm 220 according to the position of the first control part 420 and/or the position of the sliding part 430, and control the display 825 to display the opening angle of the outer clamp arm 220. In some embodiments, the display 825, the processor 823, and the first sensor 821 may be disposed on the control handle 300. In some alternative embodiments, the display 825 and/or processor 823 may be disposed away from the control handle 300. For example, the first sensor 821 may be in signal connection (such as electrical connection, wireless communication connection) with the processor 823, the first sensor 821 may send detected data to the processor 823, and the processor 823 may control the display 825 (such as a surgical monitoring display) to display the opening angle of the outer clamp arm 220 according to a processing result. By adopting the first sensor 821 and the display 825, the opening angle of the outer clamp arm 220 can be displayed more accurately, which can effectively avoid inaccurate reading caused by the visual error of the operator.

In some embodiments, the first sliding groove(s) 412 may be disposed on the sleeve 410 along its length direction. The sliding part 430 may pass through the first sliding groove(s) 412 and be connected to the first control part 420. The first sensor 821 may include a distance measuring module. The distance measuring module may be configured to detect a distance between one end of the sleeve 410 and the sliding part 430, a distance between one end of the sleeve 410 and the first control part 420, a distance between one end of the first sliding groove 412 and the sliding part 430, a distance between one end of the first sliding groove 412 and the first control part 420, or the like, or any combination thereof. The above distances may reflect the position of the first control part 420 and/or the position of the sliding part 430. Based on one or more of the above distances, the processor 823 may obtain the opening angle of the outer clamp arm 220 and control the display 825 to display it. In some embodiments, the correspondence between the position of at least one of the first control part 420 or the position of the sliding part 430 and the opening angle of the outer clamp arm 220 may be obtained by experiments. The processor 823 may determine the opening angle of the outer clamp arm 220 based on the position of the first control part and/or the position of the sliding part and the correspondence between the position of the first control part and/or the position of the sliding part and the opening angle of the outer clamp arm 220. In some embodiments, the distance measuring module may include an ultrasonic ranging sensor, an infrared distance sensor, a Hall sensor, or the like, or any combination thereof. In some embodiments, the first sensor 821 may detect the position of the first control part 420 and/or the position of the sliding part 430 in other ways. For example, the first sensor 821 may determine the position of the first control part 420 and/or the sliding part 430 by monitoring the number of turns or angles rotated by the first control part 420.

In some embodiments, the outer clamp arm control mechanism 400 may include a first prompting device 830. The first prompting device 830 may be configured to prompt that the outer clamp arm 220 has reached a preset angle of the outer clamp arm 220. Specifically, the first prompting device 830 may prompt the operator that the outer clamp arm 220 is at a specific angle (e.g., 90°, 120°, 180°, etc.) in a visual, tactile, auditory manner, etc., or any combination thereof.

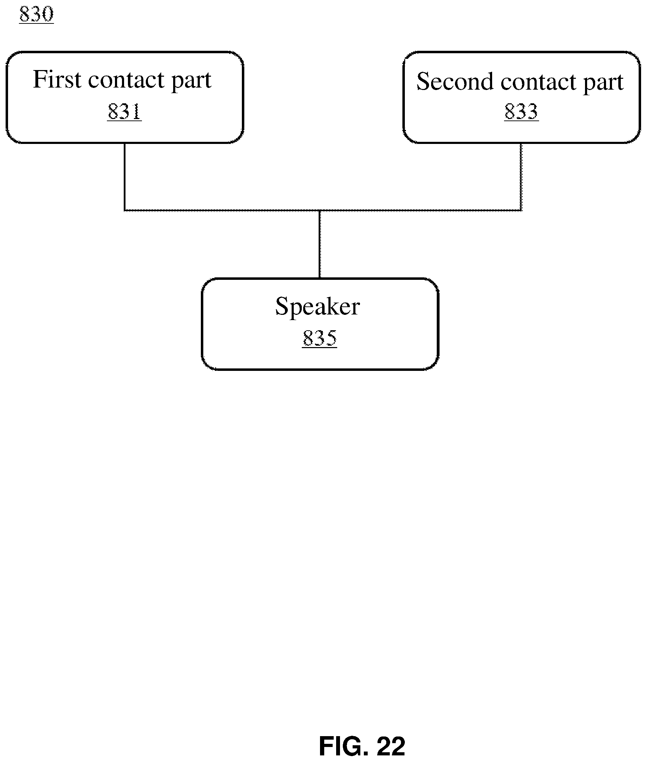

FIG. 22 is a schematic diagram of a first prompting device according to some embodiments of the present disclosure. In some embodiments, as shown in FIG. 22, the first prompting device 830 may include a first contact part 831 and a second contact part 833. The first contact part 831 may be disposed inside the sleeve 410. The second contact part 833 may be disposed outside the sliding part 430. When the second contact part 833 and the first contact part 831 are in contact, a prompt that the outer clamp arm 220 has reached the preset angle may be generated.

In some embodiments, when the first control part 420 rotates relative to the sleeve 410 and drives the sliding part 430 to move, the first contact part 831 and the second contact part 833 may be in contact or separated from each other. When the first contact part 831 and the second contact part 833 are in contact, the operator may be given a tactile and/or auditory feedback. For example, the first contact part 831 or the second contact part 833 may be a spring protrusion structure (such as a spring bead), and the other may be a corresponding recess. When the first contact part 831 and the second contact part 833 are in contact, the spring protrusion structure may be clamped in the recess, thereby giving the operator feedback of the click sound or the click feeling. In some embodiments, when the first contact part 831 and the second contact part 833 are in contact, the opening angle of the outer clamp arm 220 may reach a preset angle (e.g., 180°). By giving the operator tactile and/or auditory feedback, the operator may be effectively prompted that the outer clamp arm 220 has been opened to the preset angle.

In some embodiments, a plurality of first contact parts 831 may be disposed in the sleeve 410 and along its length direction to prompt the operator that the outer clamp arm 220 has been opened to a plurality of preset angles (e.g., 90°, 120°, 180°, etc.). Merely by way of illustration, three first contact parts 831 may be disposed in the sleeve 410, respectively corresponding to three preset angles (e.g., 90°, 120°, and 180°) of the outer clamp arm 220. The three first contact parts 831 may be spring protrusion structures. The second contact parts 833 on the sliding part 430 may be recesses. During the process of controlling the first control part 420 to rotate around the sleeve 410 to open or close the outer clamp arm 220, the three first contact parts 831 may respectively contact the second contact parts 833 to provide three tactile and/or auditory feedback. In some embodiments, in order to further distinguish the feedback at different preset angles (e.g., 90°, 120°, and 180°), the spring protrusion structures of the first contact parts 831 may be different, so that the tactile and/or auditory feedback may be different at the preset angles, and the operator can be clearly prompted.

In some embodiments, the first prompting device 830 may further include a speaker 835. In some embodiments, the speaker 835 may be disposed on the control handle 300. In some alternative embodiments, the speaker 835 may be not disposed on the control handle 300. For example, when one first contact part 831 and the second contact part 833 are in contact, the first prompting device 830 may transmit a contact signal to the speaker 835 (such as a wireless speaker) that is not disposed on the control handle 300 by a signal connection (such as an electrical connection) to give a prompt. The first contact part 831 and the second contact part 833 may include electrical contacts, contact switches, or the like. When one first contact part 831 and the second contact part 833 are in contact, the speaker 835 may play a preset voice. Exemplary preset voices may include a sound (such as a "beep" sound), angle broadcasting information (such as sound broadcasting angles), reminder broadcasting information (such as reminders for experiments or surgery), or the like, or any combination thereof. In some embodiments, when several first contact parts 831 are disposed, the speaker 835 may be configured to play one or more preset voices corresponding to the preset angles. By disposing the speaker 835, the operator can be prompted more intuitively. In some embodiments, the first prompting device 830 may further include one or more prompting components such as a light emitter, a buzzer, and a vibrator.

In some embodiments, the first prompting device 830 may simultaneously use at least two of visual, tactile, or auditory feedbacks to prompt the operator to ensure the prompt effect. Merely by way of illustration, the first contact part(s) 831 or the second contact part 833 may include a spring protrusion structure as an electrical contact. When the first contact part(s) 831 and the second contact part 833 are in contact, the first prompting device 830 may play preset voice by the speaker 835 and provide tactile feedback to the operator.

In some embodiments, a fin may be disposed on the outer circumferential surface of the sleeve 410, and a paddle corresponding to the fin may be disposed on the inner circumferential surface of the first control part 420. When the first control part 420 and the sleeve 410 rotate relatively, the paddle may move the fin on the outer circumferential surface of the sleeve 410. In some embodiments, the fin may be disposed along a spiral direction of the external thread of the sleeve 410. The fin may be made of metal foil, reeds, or other materials. When the first control part 420 drives the paddle to contact with the fin, a sound and/or a sense of frustration may be given to the operator, so that the operator may be prompted to rotate the first control part 420. By disposing the fin and paddle, the misoperation of the operator can be effectively prevented. In some alternative embodiments, a paddle may be disposed on the outer circumferential surface of the sleeve 410, and a fin may be disposed on the inner circumferential surface of the first control part 420.

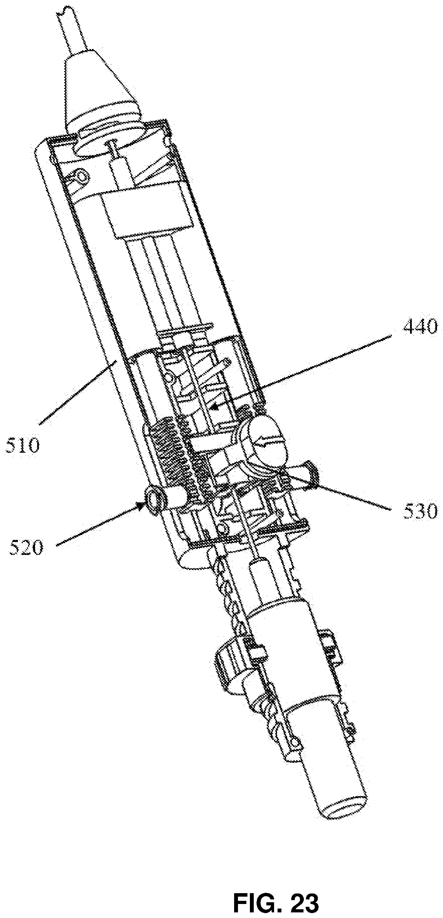

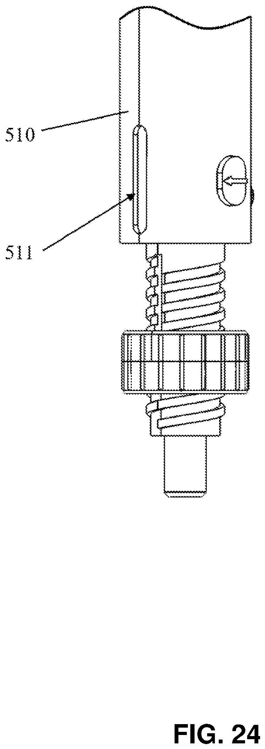

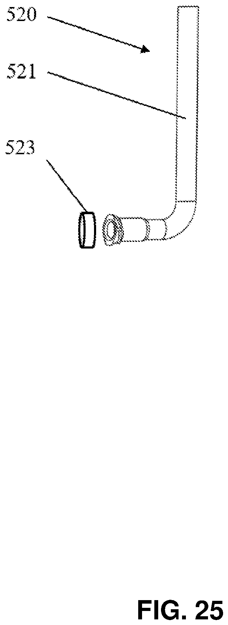

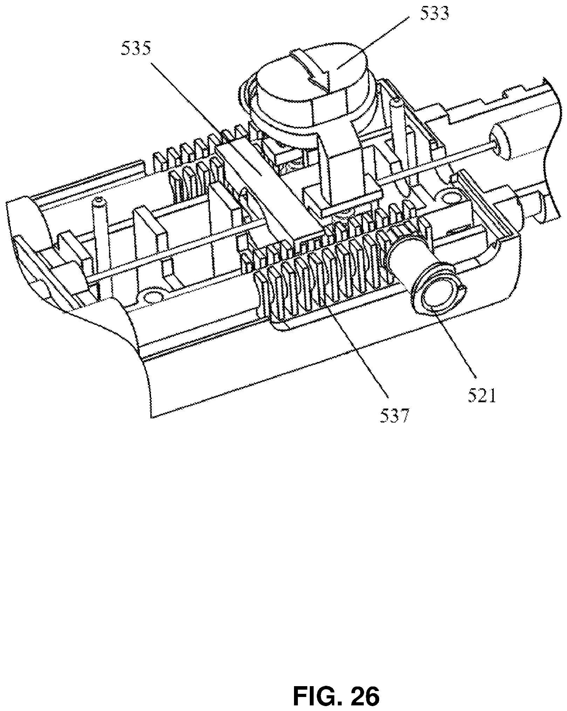

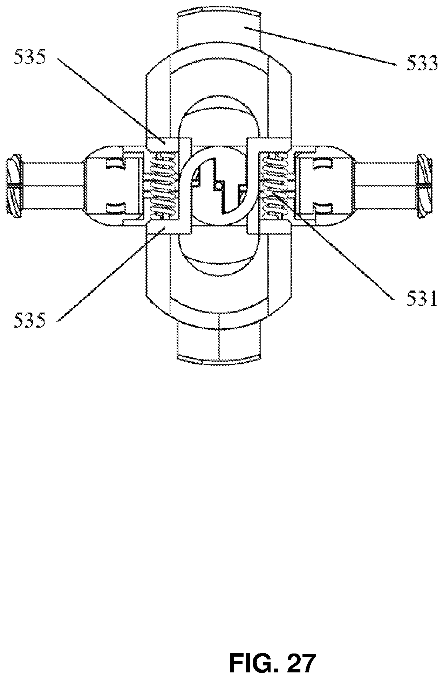

FIG. 23 is a schematic diagram of a partial cross-sectional structure of an inner clamp arm control mechanism according to some embodiments of the present disclosure. FIG. 24 is a structural schematic diagram illustrating a housing according to some embodiments of the present disclosure. FIG. 25 is a structural schematic diagram illustrating a first sub-control part according to some embodiments of the present disclosure. FIG. 26 is a structural schematic diagram illustrating the second control part and a locking mechanism at a first perspective according to some embodiments of the present disclosure. FIG. 27 is a structural schematic diagram illustrating the second control part and the locking mechanism at a second perspective according to some embodiments of the present disclosure. As shown in FIGs. 23 to 27, the inner clamp arm control mechanism 500 includes the housing 510 and a second control part 520. A second sliding groove 511 is opened on the housing 510. The second control part 520 passes through and move along the second sliding groove 511 to control the opening and closing of the inner clamp arm 210 relative to the outer clamp arm 220.

In some embodiments, as shown in FIG. 25, the second control part 520 may include an L-shaped duct 521 and an end cover 523. One end of the duct 521 may pass through the second sliding groove 511 and be detachably connected to the end cover 523. During operation, the second control part 520 may slide in the second sliding groove 511 by pushing and pulling the duct 521 at the end cover 523. By disposing the L-shaped duct 521, it is more convenient for the operator to control. In some embodiments, the second control part 520 may be drivingly connected to the inner clamp arm 210 by a traction cable. In some embodiments, the traction cable may pass through the through hole at a movable end of the inner clamp arm 210, and both ends of the traction cable may be fixed at the end cover 523. When it is necessary to separate the tissue clamping device 200 from the control handle 300, the end cover 523 and the duct 521 may be separated to release the fixing of the two ends of the traction cable and the traction cable may be drawn out, so that the control handle 300 may be separated from the inner clamp arm 210. In some embodiments, releasing the fixing of the two ends of the traction cable may include: releasing the connection between the two ends of the traction cable and the end cover 523, untying the knot formed at both ends of the traction cable, cutting the traction cable, or the like, or any combination thereof. In some embodiments, the traction cable may be incompletely removed from the control handle 300, and just detached from the inner clamp arm 210.

In some embodiments, as shown in FIG. 24, the second sliding groove 511 may be in an elongated shape. When the end cover 523 of the second control part 520 (or a portion of the duct 521 protruding from the second sliding groove 511) moves to the rear end (e.g., an end away from the tissue clamping device 200) of the second sliding groove 511, the inner clamp arm 210 may be in a closed state. In some alternative embodiments, the second sliding groove 511 may have an L-shaped contour. Specifically, a passage may be opened at the rear end of the second sliding groove 511 and along a direction that forms an angle (e.g., 90°) with the second sliding groove 511. When the end cover 523 of the second control part 520 (or the portion of the duct 521 protruding from the second sliding groove 511) moves to the rear end of the second sliding groove 511, the second control part 520 may be pushed and pulled laterally so that the part of the duct 521 protruding from of the second sliding groove is clamped into the channel. In this way, the inner clamp arm 210 be kept in a folded state (i.e., the closed state), thereby preventing misoperation during the operation.

In this embodiment, the second sliding groove 511 may include a first sub-sliding groove and a second sub-sliding groove, that are set on both sides of the housing 510. The second control part 520 may include a first sub-control part for controlling the first inner clamp arm 211 and a second sub-control part for controlling the second inner clamp arm 213. The first sub-control part and the second sub-control part may respectively correspond to separate traction cables. In some embodiments, the first sub-control part and the second sub-control part (i.e., the second control part 520) may be operated successively or simultaneously according to actual needs, so as to accurately control the first inner clamp arm 211 and the second inner clamp arm 213 (i.e., the inner clamp arm 210) according to the needs of experiments or surgery. For example, during the mitral valve repair process, the first inner clamp arm 211 may be controlled to clamp one side of the mitral valve, and then the second inner clamp arm 213 may be controlled to clamp the other side of the mitral valve. As another example, the second control part 520 may simultaneously control the first inner clamp arm 211 and the second inner clamp arm 213 to clamp the mitral valve.

In some embodiments, one end of the housing 510 may be connected (or integrally formed) with the sleeve 410, and central axes of the housing 510 and the sleeve 410 may coincide, making the control handle 300 more compact and easier to handle. For example, the housing 510 may be disposed at an end of the sleeve 410 near the tissue clamping device 200, and when the first control part 420 is in contact with the rear end of the housing 510, the outer clamp arm 220 may be in a maximum opened state. In some alternative embodiments, the housing 510 may also be disposed at the end of the sleeve 410 away from the tissue clamping device 200.

In some embodiments, as shown in FIGs. 23 to 27, the inner clamp arm control mechanism 500 may include a locking mechanism 530. The locking mechanism 530 may include a second elastic member 531, one or more locking buttons 533, and one or more locking blocks 535. The second control part 520 may further include one or more tooth-shaped connecting parts 537. The locking button(s) 533 may be configured to control the locking block(s) 535 to overcome an elastic force of the second elastic member 531, so as to release the restriction of the locking block(s) 535 on the tooth-shaped connecting part(s) 537.

As shown in FIGs. 23 to 27, in this embodiment, the locking mechanism 530 may include a pair of oppositely disposed locking blocks 535, and the first sub-control part and the second sub-control part may include tooth-shaped connecting parts 537 corresponding to the locking blocks 535, respectively. Specifically, the tooth-shaped connecting parts 537 may be connected to or integrally formed with the duct 521 of the first sub-control part and the second sub-control part, respectively. The pair of oppositely disposed locking blocks 535 may be configured to restrict the movement of the first sub-control part and the second sub-control part under the elastic force of the second elastic member 531, respectively. For example, one of the pair of locking blocks 535 corresponding to the first sub-control part may be snapped into one of the tooth-shaped connection parts 537 corresponding to the first sub-control part under the elastic force of the second elastic member 531. The other one of the pair of locking blocks 535 corresponding to the second sub-control part may be snapped into the other one of the tooth-shaped connecting parts 537 corresponding to the second sub-control part under the elastic force of the second elastic member 531. In this embodiment, the second elastic member 531 may be two springs. There may be two locking buttons 533, which are respectively connected to two locking blocks 535. The locking buttons 533 may be exposed from the housing 510. Taking the operation of the first sub-control part as an example, when one of the locking buttons 533 corresponding to the first sub-control part is pressed, it may drive the corresponding locking block 535 to overcome the elastic force of the second elastic member 531 so as to disengage from the corresponding tooth-shaped connecting part 537. The operator may push or pull the first sub-control part (such as the duct 521 of the first sub-control part) to slide in the first sub-sliding groove corresponding to the first sub-control part to control the opening and closing of the first inner clamp arm 211. When the operator releases the one of the locking buttons 533 corresponding to the first sub-control part, under the elastic force of the second elastic member 531, the locking block 535 may be re-snapped into the corresponding tooth-shaped connecting part 537 to restrict the movement of the tooth-shaped connecting part 537 (that is, the movement of the duct 521). The operation on the second sub-control part may be similar to that on the first sub-control part, which is not repeated herein.

In some embodiments, a mark may be added to each of the locking buttons 533 in order to facilitate the operator to distinguish them. The mark may include "left", "right", "L", "R", an arrow, or the like, or any combination thereof.

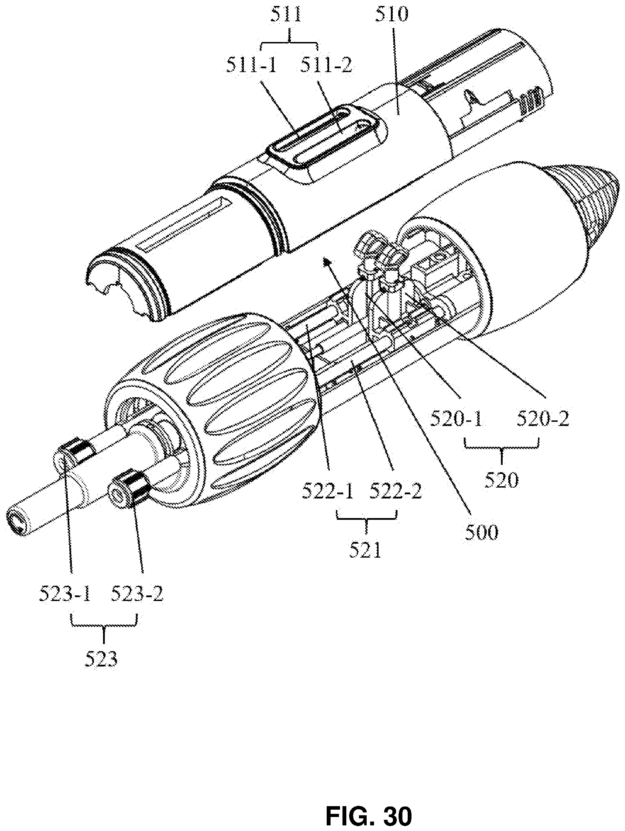

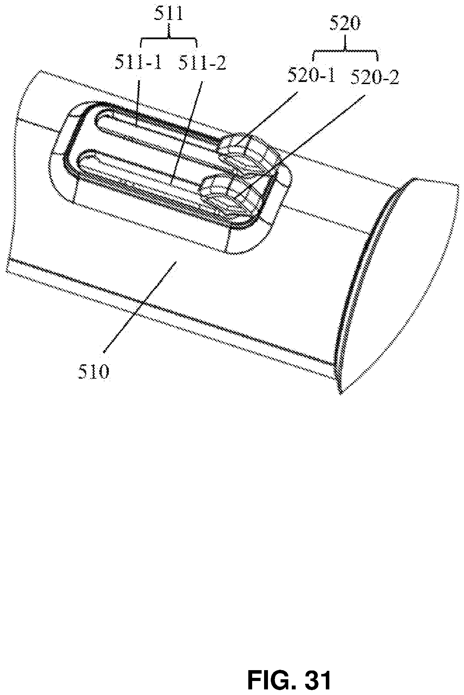

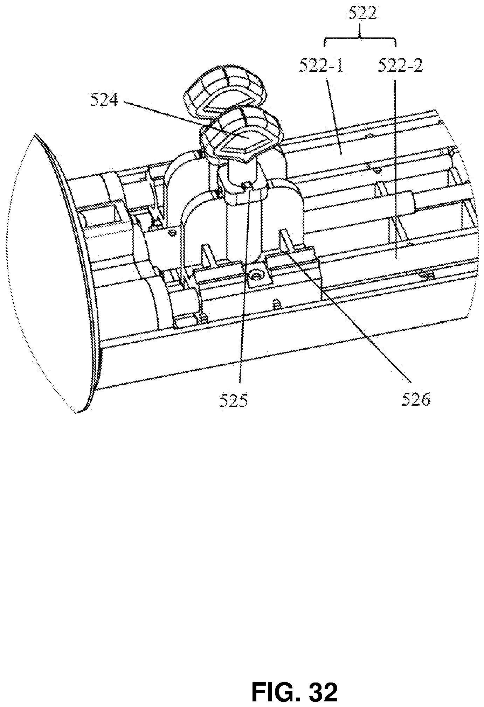

FIG. 30 is a structural schematic diagram illustrating an inner clamp arm control mechanism according to another embodiment of the present disclosure. FIG. 31 is a partial structural diagram illustrating the inner clamp arm control mechanism according to another embodiment of the present disclosure. FIG. 32 is a structural schematic diagram illustrating a second control part according to another embodiment of the present disclosure. FIG. 33 is a partial sectional diagram illustrating the inner clamp arm control mechanism according to another embodiment of the present disclosure. In some embodiments, as shown in FIGs. 30 to 33, an inner clamp arm control mechanism 500 may include a housing 510, a second control part 520, and a connecting rod 522. A second sliding groove 511 may be opened on the housing 510. The second control part 520 may pass through the second sliding groove 511 and be fixedly connected to an outer wall of the connecting rod 522. The second control part 520 may move along the second sliding groove 511 to drive the connecting rod 522 to move. The movement of the connecting rod 522 may then control the movement of the inner clamp arm 210 of the tissue clamping device 200.

In some embodiments, the connecting rod 522 may control the movement of the inner clamp arm 210 by a traction cable. In a specific embodiment, the connecting rod 522 may include an inner hole, and the traction cable may pass through the inner hole of the connecting rod 522. The traction cable may pass through the through hole at the movable end of the inner clamp arm 210. Both ends of the traction cable may be detachably connected to a rear end (e.g., an end away from the tissue clamping device 200) of the connecting rod 522. Specifically, an end cover 523 may be disposed at the rear end of the connecting rod 522, and both ends of the traction cable may be fixed at the end cover 523. For example, the end cover 523 may be connected to the connecting rod 522 by threads, and both ends of the traction cable may be fixedly locked at positions of the threaded connection between the end cover 523 and the connecting rod 522. When the tissue clamping device 200 and the control handle 300 need to be separated, the end cover 523 and the connecting rod 522 may be separated to release the fixing of the two ends of the traction cable and the traction cable may be drawn out, so that the control handle 300 may be separated from the inner clamp arm 210. In some embodiments, releasing the fixing of the two ends of the traction cable may include: releasing the connection between the two ends of the traction cable and the end cover, untying the knot formed at both ends of the traction cable, cutting the traction cable, or the like, or any combination thereof. In some embodiments, the traction cable may be incompletely removed from the control handle 300, and just detached from the inner clamp arm 210. By disposing the connecting rod 522 and the end cover 523, it is convenient for pulling of the traction cable and manufacturing the inner clamp arm control mechanism 500.

In some embodiments, the second control part 520 may include a first sub-control part 520-1 for controlling the first inner clamp arm 211 and a second sub-control part 520-2 for controlling the second inner clamp arm 213. The second sliding groove 511 may include a first sub-sliding groove 511-1 and a second sub-sliding groove 511-2 corresponding to the first sub-control part 520-1 and the second sub-control part 520-2, respectively. The connecting rod 522 may include a first sub-connecting rod 522-1 and a second sub-connecting rod 522-2 fixed to the first sub controlling part 520-1 and the second sub controlling part 520-2, respectively. A first sub-end cover 523-1 and a second sub-end cover 523-2 may be disposed at rear ends (e.g., ends away from the tissue clamping device 200) of the first sub-connecting rod 522-1 and the second sub-connecting rod 522-2, respectively. In some embodiments, as shown in FIG. 31, the first sub-sliding groove 511-1 and the second sub-sliding groove 511-2 may be disposed on the same side of the housing 510. Both the first sub-sliding groove 511-1 and the second sub-sliding groove 511-2 may be disposed in an elongated shape. In some alternative embodiments, the first sub-sliding groove 511-1 and the second sub-sliding groove 511-2 may be disposed on the housing 510 in other ways. For example, the first sub-sliding groove 511-1 and the second sub-sliding groove 511-2 may be disposed on opposite sides of the housing 510. The first sub-control part 520-1 and the second sub-control part 520-2 may move in the first sub-sliding groove 511-1 and the second sub-sliding groove 511-2, respectively, thereby driving the first sub-connecting rod 522-1 and the second sub-connecting rod 522-2 to move, respectively. The first sub-connecting rod 522-1 and the second sub-connecting rod 522-2 may respectively control the traction cables (such as the first traction cable and the second traction cable), thereby realizing the control of the first inner clamp arm 211 and the second inner clamp arm 213 of the inner clamp arm 210, respectively. Taking the first traction cable as an example, the first traction cable may pass through the through hole at the movable end of the first inner clamp arm 211, and pass through the inner hole of the first sub-connection rod 522-1. Both ends of the first traction cable may be fixed at the first sub-end cover 523-1.

In some embodiments, the second control part 520 (such as the first sub-control part 520-1 or the second sub-control part 520-2) may include a control button 524 and a connecting body 526. The control button 524 may be connected (e.g., fixedly connected or movably connected) to the connecting body 526 by passing through the second sliding groove 511. The connecting body 526 may be fixedly connected to the outer wall of the connecting rod 522 in a manner such as glue connection, welding connection, or interference connection.

In some embodiments, the control button 524 may be movably connected to the connecting body 526 by a spring (not shown in the figure). For example, a blind hole may be disposed on the connecting body 526, a connecting shaft capable of telescoping in the blind hole may be disposed at one end of the control button 524, and the end of the connecting shaft may be connected to the blind hole by a spring. The other end of the control button 524 may be exposed from the housing 510 by passing through the second sliding groove 511. There may be one or more grooves 527 and one or more corresponding protrusion blocks 525. In some embodiments, as shown in FIGs. 32 and 33, a plurality of protrusion blocks 525 may be disposed on the control button 524, and a plurality of grooves 527 may be disposed on the housing 510. The protrusion blocks 525 may be engaged with the grooves 527 under the action of the spring to restrict the movement of the second control part 520 relative to the second sliding groove 511. When needing to control the inner clamp arm 210, the operator may press the control button 524 to make the protrusion blocks 525 disengage from the grooves 527 against an elastic force of the spring, and then push and pull the control button 524 to control the second control part 520 to move relative to the second sliding groove 511. When the second control part 520 (i.e., the first sub-control part 520-1 and the second sub-control part 520-2) has been moved to be located at the corresponding positions of the grooves 527, the operator may release the control button 524, and the protrusion blocks 525 may snap into the grooves 527 under the elastic force of the spring, thereby restricting the movement of the second control part 520 relative to the second sliding groove 511. By disposing the grooves 527 and the protrusion blocks 525 together, the misoperation of the operator can be effectively prevented.

In some embodiments, the housing 510 may include two grooves 527 disposed at both ends of the second sliding groove 511, respectively. When the two protrusion blocks 525 are respectively engaged with the two grooves 527, the second control part 520 (i.e., the first sub-control part 520-1 and the second sub-control part 520-2) may be located at the two ends of the second sliding groove 511, respectively. When the second control part 520 is located at the rear end of the second sliding groove 511, the inner clamp arm 210 may be in a folded state (or closed state). When the second control part 520 is located at the front end of the second sliding groove 511, the inner clamp arm 210 may be in the maximum expandable (or opened) state. The expandable state may depend on the state of the outer clamp arm 220. By only disposing two grooves 527 at both ends of the second sliding groove 511, it is more convenient for the operator to use on the basis of surgery needs, and it is possible to further prevent the operator from misoperation during the operation. In some alternative embodiments, a plurality of grooves 527 (e.g., 4, 5, 6) may be disposed on the housing 510, so that the second control part 520 may be restricted at a plurality of positions, which is beneficial to the operator for performing more precise control.

In some embodiments, the first sub-control part 520-1 and the second sub-control part 520-2 may be configured to be operated successively or simultaneously according to actual needs, thereby achieving precise control of the inner clamp arm 210 according to the needs of experiments or surgery. For example, during the mitral valve repair process, the first inner clamp arm 211 may be first controlled to clamp one side of the mitral valve, and then the second inner clamp arm 213 may be controlled to clamp the other side of the mitral valve. As another example, the second control part 520 may simultaneously control the first inner clamp arm 211 and the second inner clamp arm 213 to clamp the mitral valve. In some embodiments, in order to facilitate the operator to recognize the first sub-control part 520-1 and the second sub-control part 520-2, a mark may be added on the control button 524. The mark may include "left", "right", "L", "R", an arrow, or the like, or any combination thereof.

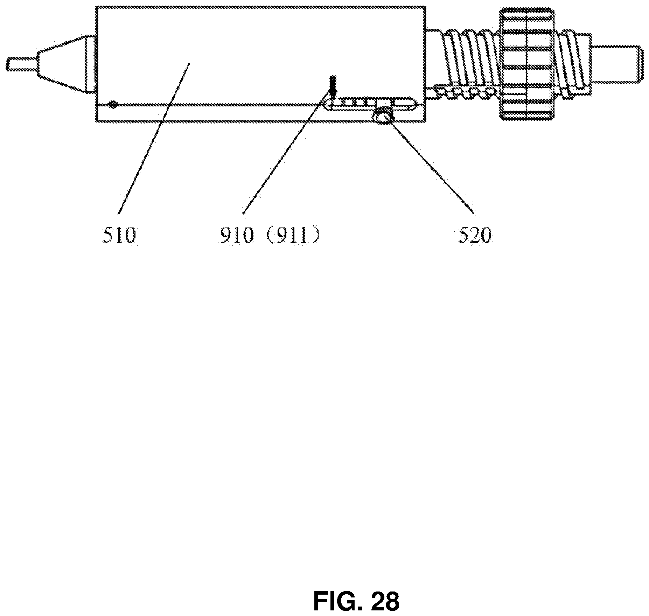

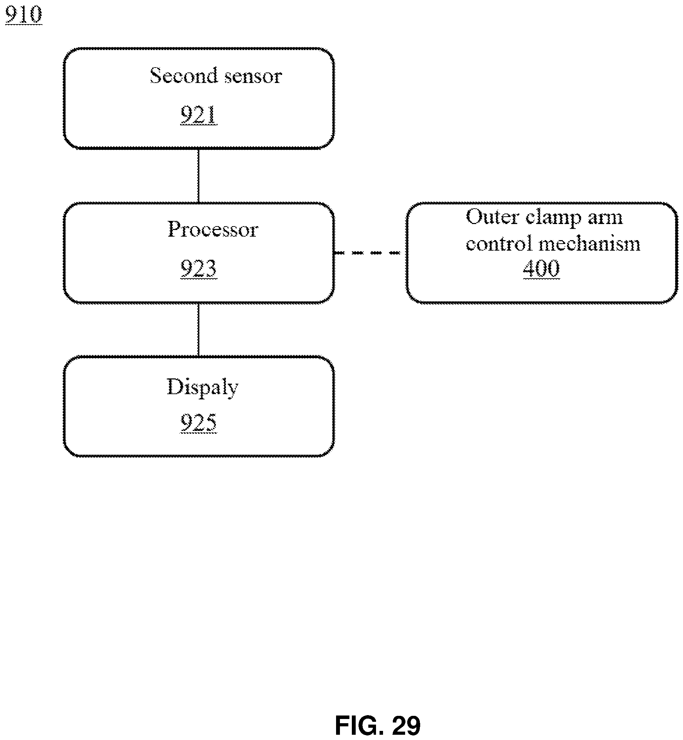

In some embodiments, the inner clamp arm control mechanism 500 may include a second indicating device 910. The second indicating device 910 may be configured to indicate an opening angle of the inner clamp arm 210 based on the position of the second control part 520. FIG. 28 is a structural schematic diagram illustrating an inner clamp arm control mechanism which can indicate an opening angle of the inner clamp arm according to some embodiments of the present disclosure. FIG. 29 is a schematic diagram illustrating a second indicating device according to some embodiments of the present disclosure. By disposing the second indicating device 910, the operator can more conveniently and intuitively control the opening angle of the inner clamp arm 210 during experiments or surgery.

In some embodiments, the opening angle of the inner clamp arm 210 may be an angle of the inner clamp arm 210 with respect to the closed state thereof. For example, the angle of the inner clamp arm 210 with respect to the closed state thereof may be an angle of the inner clamp arm 210 with respect to the supporting part 240 as shown in FIG. 2. As another example, the angle of the inner clamp arm 210 with respect to the closed state thereof may be an angle of the inner clamp arm 210 with respect to the connection pipe 270 as shown in FIG. 6. In some embodiments, the opening angle of the inner clamp arm 210 with respect to the closed state may include an opening angle of the first inner clamp arm 211 (also referred to as first opening angle in short) with respect to the closed state of the first inner clamp arm 211 and an opening angle of the second inner clamp arm 213 (also referred to as second opening angle in short) with respect to the closed state of the second inner clamp arm 213. In some embodiments, the first opening angle and the second opening angle may be the same or different. In some alternative embodiments, the opening angle of the inner clamp arm 210 may be an angle between the two inner clamp arms opening to each other. The second indicating device 910 may indicate the first and/or second opening angle of the inner clamp arm 210 based on the position of the second control part 520.

In some embodiments, as shown in FIG. 28, the second indicating device 910 may include one or more angle marks 911 disposed on the housing 510. As shown in FIG. 28, the angle mark(s) 911 may be mark(s) (such as an arrow) printed on the housing 510. The second control part 520 (such as a portion of the duct 521 protruding from the second sliding groove 511) moves to align with the angle mark(s) 911, indicating that the inner clamp arm 210 is opened to an angle the angle mark(s) 911 represent. In some embodiments, the angle mark(s) 911 may include other mark forms. For example, the angle mark(s) 911 may include a scale mark, an angle value, an indicator arrow in different colors printed on or engraved on the housing 510, etc. In some embodiments, the angle mark(s) 911 may be sequentially disposed along a length direction of the housing 510. Each of the angle mark(s) 911 may correspond to an angle (such as 0°, 30°, 60°, 90°, etc.) at which the inner clamp arm 210 is opened. In some embodiments, the corresponding relationship between each of the angle mark(s) 911 and the angle at which the inner clamp arm 210 is opened may be obtained by experiments. In some embodiments, the installation position of each component may be adjusted according to the angle corresponding to each of the angle mark(s) 911 when the inner clamp arm control mechanism 500 is to be installed. In this way, when the installation is completed, each angle mark 911 may correspond to the angle at which the inner clamp arm 210 is opened.

In some embodiments, as shown in FIG. 29, the second indicating device 910 may include a processor 923, a display 925, and a second sensor 921. The second sensor 921 may be configured to detect the position of the second control part 520. The processor 923 may be configured to determine the opening angle of the inner clamp arm 210 (e.g., the opening angle of the inner clamp arm 210 with respect to the closed state) according to the position of the second control part 520 and control the display 925 to display the opening angle of the inner clamp arm 210. In some embodiments, the display 925, the processor 923, and the second sensor 921 may be directly disposed on the control handle 300. In some alternative embodiments, the display 925 and/or the processor 923 may be disposed away from the control handle 300. For example, the second sensor 921 may be in signal connection (such as electrical connection, wireless communication connection) with the processor 923, the second sensor 921 may send detected data to the processor 923, and the processor 923 may control the display 925 (such as a surgical monitoring display) to display the opening angle of the inner clamp arm 210 according to a processing result. By using the second sensor 921 and the display 925, the opening angle of the inner clamp arm 210 can be displayed more accurately, which can effectively avoid inaccurate reading caused by the visual error of the operator. In some embodiments, the outer clamp arm control mechanism 400 and the inner clamp arm control mechanism 500 may use the same processor and/or the same display.

In some embodiments, the second sensor 921 may include a distance measuring module. The distance measuring module may be configured to detect the distance between one end of the second sliding groove 511 and the corresponding second control part (such as the portion of the duct 521 protruding from the second sliding groove 511). This distance may reflect the position of the second control part 520. In some embodiments, the correspondence between the position of the second control part 520 and the opening angle of the inner clamp arm 210 may be obtained by experiments. The processor 923 may determine the opening angle of the inner clamp arm 210 based on the position of the second control part 520 and the correspondence between the position of the second control part 520 and the opening angle of the inner clamp arm 210. In some embodiments, the distance measuring module may include an ultrasonic ranging sensor, an infrared distance sensor, a Hall sensor, or the like, or any combination thereof.

In some embodiments, the opening angle of the inner clamp arm 210 (or referred to as third opening angle in short) may be an angle of the inner clamp arm 210 with respect to the outer clamp arm 220, that is, an angle of the first inner clamp arm 211 with respect to the corresponding first outer clamp arm 221 or an angle of the second inner clamp arm 213 with respect to the corresponding second outer clamp arm 223. It can be understood that the opening angle of the inner clamp arm 210 with respect to the outer clamp arm 220 may not be directly indicated in some embodiments, and may be determined by the opening angle of the inner clamp arm 210 and the opening angle of the outer clamp arm 220. In some embodiments, the second indicating device 910 may be configured to indicate the third opening angle of the inner clamp arm 210 according to the position of the second control part 520 and the opening angle of the outer clamp arm 220. The opening angle of the outer clamp arm 220 may be determined by the outer clamp arm control mechanism 400. In some embodiments, as shown in FIG. 29, the processor 923 may be configured to determine the third opening angle of the inner clamp arm 210 according to the position of the second control part 520 and the opening angle of the outer clamp arm 220, and control the display 925 to display it. For example, the processor 923 may obtain the opening angle of the outer clamp arm 220 from the processor 823. As another example, the processor 923 may obtain the opening angle of the outer clamp arm 220 according to the data detected by the first sensor 821. In this embodiment of the present disclosure, the opening angle of the outer clamp arm 220 may be understood as the angle between the two outer clamp arms opening to each other, and the processor 923 may determine the opening angle (e.g., the first opening angle or the second opening angle) of the inner clamp arm 210 according to the position of the second control part 520. The processor 923 may determine the third opening angle of the inner clamp arm by arithmetic operations. For example, the opening angle of the outer clamp arm 220 obtained by the processor 923 from the outer clamp arm control mechanism 400 may be 180°, and a first opening angle of the first inner clamp arm 211 by the processor 923 based on data obtained from the second sensor 921 (e.g., the position of the second control part 520) is 30°. Then, the processor 923 may determine that the third opening angle of the inner clamp arm 210 (i.e., an angle of the first inner clamp arm 211 with respect to the corresponding first outer clamp arm 221) is 60°, that is determined by an equation of (180°÷2)-30°. It should be noted that, in some embodiments, when the first opening angle or the second opening angle is greater than half of the opening angle of the outer clamp arm 220, it means that the first inner clamp arm or the second inner clamp arm is closed relative to the corresponding first outer clamp arm or the second outer clamp arm, and the traction cable corresponding to the first inner clamp arm or the second inner clamp arm is in a relaxed state.

In some embodiments, the inner clamp arm control mechanism 500 may include a second prompting device. The second prompting device may be configured to prompt the inner clamp arm 210 to reach a preset angle. Specifically, the preset angle of the inner clamp arm 210 may be a preset opening angle of the inner clamp arm 210 with respect to the closed state, or a preset opening angle of the inner clamp arm 210 with respect to the outer clamp arm 220. In some embodiments, the second prompting device may prompt the operator at a specific angle (such as 0°, 30°, 60°, 90°, etc.) in a visual, tactile, auditory manner (e.g., feedback), etc., or any combination thereof. By giving the operator visual, auditory, tactile feedback, the operator can be effectively prompted that the inner clamp arm 210 has reached the preset angle.

In some embodiments, the second prompting device may include one or more first contact parts and one or more second contact parts similar to the first prompting device 830. The first contact part(s) may be disposed on an outer circumferential surface of the second control part, and the second contact part(s) may be disposed inside the housing. When the second contact part(s) contacts the first contact part(s), respectively, the second prompting device may prompt the inner clamp arm 210 to reach the preset angle. In some embodiments, the second prompting device may include a prompting component. The prompting component may include, but is not limited to, speakers, light emitters, buzzers, vibrators, or the like, or any combination thereof. In some embodiments, the prompting component may be controlled by the processor 923. When the processor 923 detects that the inner clamp arm 210 reaches the preset opening angle, the processor 923 may control the prompting component to issue a prompt.

In some embodiments, the second control part 520 may include a first sub-control part and a second sub-control part for controlling the opening and closing of the first inner clamp arm 211 and the second inner clamp arm 213, respectively. The second indicating device 910 may include a first sub-indicating device and a second sub-indicating device for indicating the opening angle of the first inner clamp arm 211 (i.e., the first opening angle) and the opening angle of the second inner clamp arm 213 (i.e., the second opening angle), respectively. The second prompting device may include a first sub-prompting device and a second sub-prompting device for prompting the first inner clamp arm 211 and the second inner clamp arm 213 to reach preset angles, respectively.

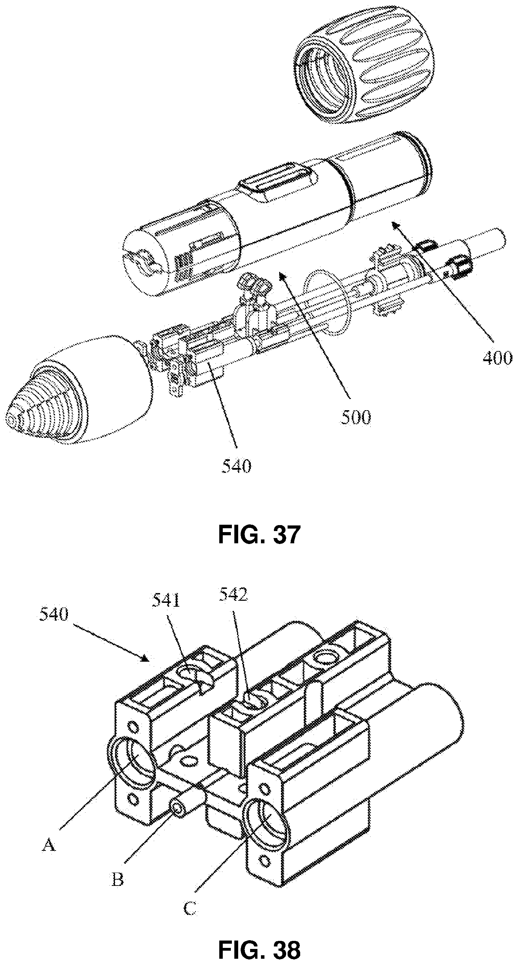

FIG. 37 is a structural schematic diagram illustrating a control handle according to another embodiment of the present disclosure. FIG. 38 is a structural schematic diagram illustrating a guiding block according to another embodiment of the present disclosure. In some embodiments, a control handle 300 may further include a guide block 540 as shown in FIGs. 37 and 38. The guide block 540 may include three spaced through holes A, B, and C, central axes of which are located on a first plane. The first plane may be the plane where the central axes of the through holes A, B and C are located. In some embodiments, the through holes A and C may be located on both sides of the guide block 540, and configured to guide the connecting rod 522 that controls the movement of the inner clamp arm 210 (e.g., the through hole A is configured to guide the first sub-connecting rod 522-1, the through hole C is configured to guide the second sub-connecting rod 522-2). The through hole B may be located in the middle of the through holes A and C, and configured to guide the driving rod 440 that controls the movement of the outer clamp arm 220. On one side of the first plane (the upper side as shown in FIG. 38), the through hole A and the through hole B may communicate with each other through a hose (not shown in the figure), and on the other side of the first plane (the lower side as shown in of FIG. 38), the through hole B and the through hole C may communicate with each other through a hose, thereby ensuring good sealing of the guide block 540. Specifically, taking the through hole A and the through hole B communicating with each other through the hose as an example, a first intersecting hole 541, which penetrates from the inside of the through hole A to a top surface of the guide block 540 shown in FIG. 38, may be disposed in the middle of the through hole A. A second intersecting hole 542, which penetrates from the inside of the through hole B to the top surface of the guide block 540 may be disposed in the middle of the through hole B. The outlets of the first intersecting hole 541 and the second intersecting hole 542 at the top surface of the guide block 540 may communicate with each other through the hose. In some embodiments, a seal member (such as a seal ring) may be disposed between a rear end (e.g., an end away from the tissue clamping device 200) of the guide block 540 (such as rear ends of the through holes A, B, and C) and the housing 510 (such as an inner baffle disposed on the housing 510) , thereby ensuring the sealing of the control handle 300, and further ensuring the smooth progress of the surgery or experiments.

The beneficial effects that the embodiments of the present disclosure may bring include but are not limited to: (1) accurately controlling the outer clamp arm and/or the inner clamp arm of the tissue clamping device; (2) quickly controlling the outer clamp arm of the tissue clamping device; (3) easily separating the control handle from the tissue clamping device; (4) making the operation of mitral valve repair more convenient, and the efficiency and the success rate of mitral valve repair improved; (5) quickly and intuitively obtaining an opening angle of the outer clamp arm and/or the inner clamp arm; (6) effectively preventing misoperation during the mitral valve repair; (7) making the control handle easy to manufacture. It should be noted that different embodiments may have different beneficial effects. In different embodiments, the possible beneficial effects may be any one or a combination of the above, or any other possible beneficial effects.

Moreover, certain terminology has been used to describe embodiments of the present disclosure. For example, the terms "one embodiment," "an embodiment," and "some embodiments" mean that a particular feature, structure or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures or characteristics may be combined as suitable in one or more embodiments of the present disclosure.

Further, it will be appreciated by one skilled in the art, aspects of the present disclosure may be illustrated and described herein in any of a number of patentable classes or context including any new and useful process, machine, manufacture, or composition of matter, or any new and useful improvement thereof. Accordingly, aspects of the present disclosure may be implemented entirely hardware, entirely software (including firmware, resident software, micro-code, etc.) or combining software and hardware implementation that may all generally be referred to herein as a "module," "unit," "component," "device," or "system." Furthermore, aspects of the present disclosure may take the form of a computer program product embodied in one or more computer-readable medium having computer readable program code embodied thereon.

Similarly, it should be appreciated that in the foregoing description of embodiments of the present disclosure, various features are sometimes grouped together in a single embodiment, figure, or description thereof to streamline the disclosure aiding in the understanding of one or more of the various embodiments.

## Claims

1. A control handle (300) for mitral valve repair, comprising:
an outer clamp arm control mechanism (400) which is configured to control a movement of an outer clamp arm (220) of a tissue clamping device (200) and includes a sleeve (410), a first control part (420), and a sliding part (430), wherein
the sliding part (430) is disposed in the sleeve (410), and
the first control part (420) rotates to drive the sliding part (430) to move in the sleeve (410) along a length direction of the sleeve (410), so as to control the opening and closing of the outer clamp arm (220); and
an inner clamp arm control mechanism (500) which is configured to control a movement of an inner clamp arm (210) of the tissue clamping device (200) and includes a housing (510) and a second control part (520), wherein
a second sliding groove (511) is set on the housing (510), and
the second control part (520) passes through and moves along the second sliding groove (511) to control the opening and closing of the inner clamp arm (210) relative to the outer clamp arm (220);
wherein the outer clamp arm control mechanism (400) further includes a driving rod (440), a fixing block (460), and a protective cover (470);
the sliding part (430) controls the opening and closing of the outer clamp arm (220) by the driving rod (440);
a rear end of the driving rod (440) is fixedly connected to the fixing block (460);
the protective cover (470) is detachably connected to the sliding part (430) by a thread; and
when connected to the sliding part (430), the protective cover (470) restricts a relative movement of the fixing block (460) and the sliding part (430).

2. The control handle (300) according to claim 1, wherein
an external thread is disposed on an outer circumferential surface of the sleeve (410);
an internal thread is disposed on an inner circumferential surface of the first control part (420);
the sleeve (410) and the first control part (420) are connected by the external thread and the internal thread;
a first sliding groove (412) is set on the sleeve (410) and along the length direction of the sleeve (410); and
the sliding part (430) passes through the first sliding groove (412) and is connected to the first control part (420).

3. The control handle (300) according to claim 2, wherein
the first control part (420) includes a thread engagement mechanism (450);
the thread engagement mechanism (450) includes manipulation buttons (451), a first elastic member (453), and a pair of engagement members (455);
the engagement members (455) are symmetrically disposed and configured to be engaged with the external thread of the sleeve (410) by an elastic force of the first elastic member (453); and
the manipulation button (451) is disposed on an outer side of the engagement members (455) and configured to control the engagement members (455) to be separated from the external thread of the sleeve (410) by overcoming the elastic force of the first elastic member (453).

4. The control handle (300) according to claim 1, wherein
the sliding part (430) comprises an outer cover (431), a middle tube (433), and an inner pipe (435);
the middle tube (433) is sleeved outside the inner pipe (435);
one or more openings (434) are set on the middle tube (433); and
one or more fixture blocks (437) that can extend outward are disposed on the inner pipe (435), and engaged with the outer cover (431) by passing through the one or more openings (434), so as to restrict a relative movement of the middle tube (433), the inner pipe (435) and the outer cover (431) in the length direction of the sleeve (410).

5. The control handle (300) according to claim 4, wherein
one or more locking teeth (439) are disposed at a front end of the middle tube (433);
one or more locking grooves (438) corresponding to the one or more locking teeth (439) are disposed at the outer cover (431); and
the one or more locking teeth (439) are engaged with the one or more locking grooves (438) to restrict a relative rotation of the middle tube (433) and the outer cover (431).

6. The control handle (300) according to claim 5, wherein a rear end of the middle tube (433) is disposed with a boss (480), and a rear end of the inner pipe (435) extends from the middle tube (433); and
by at least one of pulling the boss (480) or pushing the rear end of the inner pipe (435), the one or more locking teeth (439) are separated from the one or more locking grooves (438), and the one or more fixture blocks (437) are pressed into the middle tube (433).

7. The control handle (300) according to claim 1, wherein the second control part (520) includes an L-shaped duct (521) and an end cover (523); and one end of the duct (521) passes through the second sliding groove (511) and is detachably connected to the end cover (523).

8. The control handle (300) according to claim 1, wherein the second control part (520) includes a first sub-control part (520-1) for controlling a first inner clamp arm (211) and a second sub-control part (520-2) for controlling a second inner clamp arm (213).

9. The control handle (300) according to claim 1, wherein
the inner clamp arm control mechanism (500) further includes a locking mechanism (530);
the locking mechanism (530) includes a second elastic member (531), one or more locking buttons (533), and one or more locking blocks (535);
the second control part (520) further includes one or more tooth-shaped connecting parts (537); and
the one or more locking buttons (533) are configured to control the one or more locking blocks (535) to overcome an elastic force of the second elastic member (531), so as to release the restriction of the one or more locking blocks (535) on the one or more toothed-shaped connecting parts (537).

10. The control handle (300) according to claim 1, further comprising a flexible tube control mechanism (620) that includes a screw (621), a rotating part (622), and a traction part (623), wherein
the screw (621) and the traction part (623) are threadedly connected;
the rotating part (622) is configured to drive the screw (621) to rotate, thereby driving the traction part (623) to move; and
the movement of the traction part (623) controls a flexible tube (610) to bend.

11. The control handle (300) according to claim 1, wherein the outer clamp arm control mechanism (400) further includes a first indicating device (810), the first indicating device (810) being configured to indicate an opening angle of the outer clamp arm (220) according to a position of at least one of the first control part (420) or the sliding part (430).

12. The control handle (300) according to claim 11, wherein the first indicating device (810) includes one or more angle marks (811) disposed on the sleeve (410).

13. The control handle (300) according to claim 11, wherein
the first indicating device (810) includes a processor (823), a display (825), and a first sensor (821);
the first sensor (821) is configured to detect the position of at least one of the first control part (420) or the sliding part (430); and
the processor (823) is configured to determine the opening angle of the outer clamp arm (220) according to the position of at least one of the first control part (420) or the sliding part (430), and control the display (825) to display the opening angle of the outer clamp arm (220).

14. The control handle (300) according to claim 1, wherein the inner clamp arm control mechanism (500) further includes a second indicating device (910), the second indicating device (910) being configured to indicate an opening angle of the inner clamp arm (210) based on a position of the second control part (520).

15. A mitral valve repair device (100), comprising a tissue clamping device (200) and a control handle (300) of any one of claims 1-14, wherein
the tissue clamping device (200) includes an outer clamp arm (220) and an inner clamp arm (210).

## Patentansprüche

1. Steuergriff (300) zur Mitralklappenreparatur, umfassend:
einen äußeren Klemmarm-Steuermechanismus (400), der konfiguriert ist, um eine Bewegung eines äußeren Klemmarms (220) einer Gewebeklemmvorrichtung (200) zu steuern, und eine Hülse (410), ein erstes Steuerteil (420) und ein Gleitteil (430) beinhaltet, wobei
das Gleitteil (430) in der Hülse (410) angeordnet ist, und
das erste Steuerteil (420) sich dreht, um das Gleitteil (430) anzutreiben, sich in der Hülse (410) entlang einer Längsrichtung der Hülse (410) zu bewegen, um das Öffnen und Schließen des äußeren Klemmarms (220) zu steuern; und
einen inneren Klemmarm-Steuermechanismus (500), der konfiguriert ist, um eine Bewegung eines inneren Klemmarms (210) der Gewebeklemmvorrichtung (200) zu steuern, und ein Gehäuse (510) und ein zweites Steuerteil (520) beinhaltet, wobei
eine zweite Gleitnut (511) am Gehäuse (510) eingerichtet ist, und
das zweite Steuerteil (520) durch die zweite Gleitnut (511) verläuft und sich entlang dieser bewegt, um das Öffnen und Schließen des inneren Klemmarms (210) in Bezug zum äußeren Klemmarm (220) zu steuern;
wobei der äußere Klemmarm-Steuermechanismus (400) weiter eine Antriebsstange (440), einen Befestigungsblock (460) und eine Schutzabdeckung (470) beinhaltet;
das Gleitteil (430) das Öffnen und Schließen des äußeren Klemmarms (220) durch die Antriebsstange (440) steuert;
ein hinteres Ende der Antriebsstange (440) fest mit dem Befestigungsblock (460) verbunden ist;
die Schutzabdeckung (470) durch ein Gewinde lösbar mit dem Gleitteil (430) verbunden ist; und
wenn mit dem Gleitteil (430) verbundenen, die Schutzabdeckung (470) eine relative Bewegung des Befestigungsblocks (460) und des Gleitteils (430) zueinander einschränkt.

2. Steuergriff (300) nach Anspruch 1, wobei
ein Außengewinde an einer Außenumfangsoberfläche der Hülse (410) angeordnet ist;
ein Innengewinde an einer Innenumfangsoberfläche des ersten Steuerteils (420) angeordnet ist;
die Hülse (410) und das erste Steuerteil (420) durch das Außengewinde und das Innengewinde verbunden sind;
eine erste Gleitnut (412) an der Hülse (410) und entlang der Längsrichtung der Hülse (410) eingerichtet ist; und
das Gleitteil (430) durch die erste Gleitnut (412) hindurch verläuft und mit dem ersten Steuerteil (420) verbunden ist.

3. Steuergriff (300) nach Anspruch 2, wobei
das erste Steuerteil (420) einen Gewindeeingriffsmechanismus (450) beinhaltet;
der Gewindeeingriffsmechanismus (450) Manipulationsknöpfe (451), ein erstes elastisches Element (453) und ein Paar Eingriffselemente (455) beinhaltet;
die Eingriffselemente (455) symmetrisch angeordnet und konfiguriert sind, um durch eine elastische Kraft des ersten elastischen Elements (453) mit dem Außengewinde der Hülse (410) in Eingriff gebracht zu werden; und
der Manipulationsknopf (451) an einer Außenseite der Eingriffselemente (455) angeordnet ist und konfiguriert ist, um die Eingriffselemente (455) zu steuern, um durch Überwinden der elastischen Kraft des ersten elastischen Elements (453) von dem Außengewinde der Hülse (410) getrennt zu werden.

4. Steuergriff (300) nach Anspruch 1, wobei
das Gleitteil (430) eine Außenabdeckung (431), ein Mittelrohr (433) und ein Innenrohr (435) umfasst;
das Mittelrohr (433) das Innenrohr (435) außen umhüllt;
eine oder mehrere Öffnungen (434) am Mittelrohr (433) eingerichtet sind; und
ein oder mehrere Befestigungsblöcke (437), die sich nach außen erstrecken können, am Innenrohr (435) angeordnet sind und mit der Außenabdeckung (431) in Eingriff stehen, indem sie durch die eine oder mehrere Öffnungen (434) hindurchlaufen, um eine relative Bewegung des Mittelrohrs (433), des Innenrohrs (435) und der Außenabdeckung (431) zueinander in der Längsrichtung der Hülse (410) einzuschränken.

5. Steuergriff (300) nach Anspruch 4, wobei
ein oder mehrere Verriegelungszähne (439) an einem vorderen Ende des Mittelrohrs (433) angeordnet sind;
eine oder mehrere Verriegelungsnuten (438), die dem einen oder den mehreren Verriegelungszähnen (439) entsprechen, an der Außenabdeckung (431) angeordnet sind; und
der eine oder mehrere Verriegelungszähne (439) mit der einen oder mehreren Verriegelungsnuten (438) in Eingriff stehen, um eine relative Drehung des Mittelrohrs (433) und der Außenabdeckung (431) zueinander einzuschränken.

6. Steuergriff (300) nach Anspruch 5, wobei ein hinteres Ende des Mittelrohrs (433) mit einer Erhebung (480) angeordnet ist und sich ein hinteres Ende des Innenrohrs (435) aus dem Mittelrohr (433) erstreckt; und
durch mindestens eines von Ziehen der Erhebung (480) oder Drücken des hinteren Endes des Innenrohrs (435) der eine oder mehrere Verriegelungszähne (439) von der einen oder mehreren Verriegelungsnuten (438) getrennt werden und der eine oder mehrere Befestigungsblöcke (437) in das Mittelrohr (433) gedrückt werden.

7. Steuergriff (300) nach Anspruch 1, wobei das zweite Steuerteil (520) einen L-förmigen Kanal (521) und eine Endabdeckung (523) beinhaltet; und ein Ende des Kanals (521) durch die zweite Gleitnut (511) hindurch verläuft und lösbar mit der Endabdeckung (523) verbunden ist.

8. Steuergriff (300) nach Anspruch 1, wobei das zweite Steuerteil (520) ein erstes Untersteuerteil (520-1) zum Steuern eines ersten inneren Klemmarms (211) und ein zweites Untersteuerteil (520-2) zum Steuern eines zweiten inneren Klemmarms (213) beinhaltet.

9. Steuergriff (300) nach Anspruch 1, wobei
der innere Klemmarm-Steuermechanismus (500) weiter einen Verriegelungsmechanismus (530) beinhaltet;
der Verriegelungsmechanismus (530) ein zweites elastisches Element (531), einen oder mehrere Verriegelungsknöpfe (533) und einen oder mehrere Verriegelungsblöcke (535) beinhaltet;
das zweite Steuerteil (520) weiter ein oder mehrere zahnförmige Verbindungsteile (537) beinhaltet; und
der eine oder mehrere Verriegelungsknöpfe (533) konfiguriert sind, um den einen oder mehrere Verriegelungsblöcke (535) zu steuern, um eine elastische Kraft des zweiten elastischen Elements (531) zu überwinden, um die Einschränkung des einen oder der mehreren Verriegelungsblöcke (535) auf das eine oder mehrere zahnförmige Verbindungsteile (537) zu lösen.

10. Steuergriff (300) nach Anspruch 1, der weiter einen flexiblen Rohrsteuermechanismus (620) umfasst, der eine Schraube (621), ein Drehteil (622) und ein Zugteil (623) beinhaltet, wobei
die Schraube (621) und das Zugteil (623) durch ein Gewinde miteinander verbunden sind;
das Drehteil (622) konfiguriert ist, um die Schraube (621) anzutreiben, sich zu drehen, wodurch das Zugteil (623) zum Bewegen angetrieben wird; und
die Bewegung des Zugteils (623) ein flexibles Rohr (610) steuert, sich zu biegen.

11. Steuergriff (300) nach Anspruch 1, wobei der äußere Klemmarm-Steuermechanismus (400) weiter eine erste Anzeigevorrichtung (810) beinhaltet, wobei die erste Anzeigevorrichtung (810) konfiguriert ist, um einen Öffnungswinkel des äußeren Klemmarms (220) gemäß einer Position von mindestens einem von dem ersten Steuerteil (420) oder dem Gleitteil (430) anzuzeigen.

12. Steuergriff (300) nach Anspruch 11, wobei die erste Anzeigevorrichtung (810) eine oder mehrere Winkelmarkierungen (811) beinhaltet, die auf der Hülse (410) angeordnet sind.

13. Steuergriff (300) nach Anspruch 11, wobei
die erste Anzeigevorrichtung (810) einen Prozessor (823), eine Anzeige (825) und einen ersten Sensor (821) beinhaltet;
der erste Sensor (821) konfiguriert ist, um die Position von mindestens einem von dem ersten Steuerteil (420) oder dem Schiebeteil (430) zu erfassen; und
der Prozessor (823) konfiguriert ist, um den Öffnungswinkel des äußeren Klemmarms (220) gemäß der Position von mindestens einem von dem ersten Steuerteil (420) oder dem Gleitteil (430) zu bestimmen und die Anzeige (825) zu steuern, um den Öffnungswinkel des äußeren Klemmarms (220) anzuzeigen.

14. Steuergriff (300) nach Anspruch 1, wobei der innere Klemmarm-Steuermechanismus (500) weiter eine zweite Anzeigevorrichtung (910) beinhaltet, wobei die zweite Anzeigevorrichtung (910) konfiguriert ist, um einen Öffnungswinkel des inneren Klemmarms (210) basierend auf einer Position des zweiten Steuerteils (520) anzuzeigen.

15. Mitralklappenreparaturvorrichtung (100), die eine Gewebeklemmvorrichtung (200) und einen Steuergriff (300) nach einem der Ansprüche 1-14 umfasst, wobei
die Gewebeklemmvorrichtung (200) einen äußeren Klemmarm (220) und einen inneren Klemmarm (210) beinhaltet.

## Revendications

1. Poignée de commande (300) pour une réparation de valvule mitrale, comprenant :
un mécanisme de commande de bras de serrage externe (400) qui est configuré pour commander un mouvement d'un bras de serrage externe (220) d'un dispositif de serrage de tissu (200) et inclut un manchon (410), une première partie de commande (420) et un partie coulissante (430), dans laquelle
la partie coulissante (430) est disposée dans le manchon (410), et
la première partie de commande (420) tourne pour entraîner la partie coulissante (430) à se déplacer dans le manchon (410) le long d'une direction longitudinale du manchon (410) de manière à commander l'ouverture et la fermeture du bras de serrage externe (220) ; et
un mécanisme de commande de bras de serrage interne (500) qui est configuré pour commander un mouvement d'un bras de serrage interne (210) du dispositif de serrage de tissu (200) et inclut un boîtier (510) et une seconde partie de commande (520), dans laquelle
une seconde rainure de coulissement (511) est ménagée sur le boîtier (510), et
la seconde partie de commande (520) traverse et se déplace le long de la seconde rainure de coulissement (511) pour commander l'ouverture et la fermeture du bras de serrage interne (210) par rapport au bras de serrage externe (220) ;
dans laquelle le mécanisme de commande de bras de serrage externe (400) inclut en outre une tige d'entraînement (440), un bloc de fixation (460) et un couvercle de protection (470) ;
la partie coulissante (430) commande l'ouverture et la fermeture du bras de serrage externe (220) par la tige d'entraînement (440) ;
une extrémité arrière de la tige d'entraînement (440) est reliée de manière fixe au bloc de fixation (460) ;
le couvercle de protection (470) est relié de manière amovible à la partie coulissante (430) par un filetage ; et
lorsqu'il est relié à la partie coulissante (430), le couvercle de protection (470) limite un déplacement relatif du bloc de fixation (460) et de la partie coulissante (430).

2. Poignée de commande (300) selon la revendication 1, dans laquelle
un filetage externe est disposé sur une surface circonférentielle externe du manchon (410) ;
un filetage interne est disposé sur une surface circonférentielle interne de la première partie de commande (420) ;
le manchon (410) et la première partie de commande (420) sont reliés par le filetage externe et le filetage interne ;
une première rainure de coulissement (412) est ménagée sur le manchon (410) et le long de la direction longitudinale du manchon (410) ; et
la partie coulissante (430) traverse la première rainure de coulissement (412) et est reliée à la première partie de commande (420).

3. Poignée de commande (300) selon la revendication 2, dans laquelle
la première partie de commande (420) inclut un mécanisme de mise en prise par filetage (450) ;
le mécanisme de mise en prise par filetage (450) inclut des boutons de manipulation (451), un premier élément élastique (453) et une paire d'éléments de mise en prise (455) ;
les éléments de mise en prise (455) sont disposés symétriquement et configurés pour être mis en prise avec le filetage externe du manchon (410) par l'intermédiaire d'une force élastique du premier élément élastique (453) ; et
le bouton de manipulation (451) est disposé sur un côté extérieur des éléments de mise en prise (455) et configuré pour commander aux éléments de mise en prise (455) d'être séparés du filetage externe du manchon (410) en surmontant la force élastique du premier élément élastique (453).

4. Poignée de commande (300) selon la revendication 1, dans laquelle
la partie coulissante (430) comprend un couvercle externe (431), un tube médian (433) et un tuyau interne (435) ;
le tube médian (433) est manchonné à l'extérieur du tuyau interne (435) ;
une ou plusieurs ouvertures (434) sont ménagées sur le tube médian (433) ; et
un ou plusieurs blocs de fixation (437) qui peuvent s'étendre vers l'extérieur sont disposés sur le tuyau interne (435) et sont mis en prise avec le couvercle externe (431) en passant à travers les une ou plusieurs ouvertures (434), de manière à limiter un mouvement relatif du tube médian (433), du tuyau interne (435) et du couvercle externe (431) dans la direction longitudinale du manchon (410).

5. Poignée de commande (300) selon la revendication 4, dans laquelle
une ou plusieurs dents de verrouillage (439) sont disposées au niveau d'une extrémité avant du tube médian (433) ;
une ou plusieurs rainures de verrouillage (438) correspondant aux une ou plusieurs dents de verrouillage (439) sont disposées au niveau du couvercle externe (431) ; et
les une ou plusieurs dents de verrouillage (439) sont mises en prise avec les une ou plusieurs rainures de verrouillage (438) pour limiter une rotation relative du tube médian (433) et du couvercle externe (431).

6. Poignée de commande (300) selon la revendication 5, dans laquelle une extrémité arrière du tube médian (433) est dotée d'un bossage (480), et une extrémité arrière du tuyau interne (435) s'étend à partir du tube médian (433) ; et
en au moins tirant sur le bossage (480) et/ou en poussant l'extrémité arrière du tuyau interne (435), les une ou plusieurs dents de verrouillage (439) sont séparées des une ou plusieurs rainures de verrouillage (438), et les un ou plusieurs blocs de fixation (437) sont pressés dans le tube médian (433).

7. Poignée de commande (300) selon la revendication 1, dans laquelle la seconde partie de commande (520) inclut un conduit en forme de L (521) et un couvercle d'extrémité (523) ; et une extrémité du conduit (521) traverse la seconde rainure de coulissement (511) et est reliée de manière amovible au couvercle d'extrémité (523).

8. Poignée de commande (300) selon la revendication 1, dans laquelle la seconde partie de commande (520) inclut une première partie de commande secondaire (520-1) pour commander un premier bras de serrage interne (211) et une seconde partie de commande secondaire (520-2) pour commander un second bras de serrage interne (213).

9. Poignée de commande (300) selon la revendication 1, dans laquelle
le mécanisme de commande de bras de serrage interne (500) inclut en outre un mécanisme de verrouillage (530) ;
le mécanisme de verrouillage (530) inclut un second élément élastique (531), un ou plusieurs boutons de verrouillage (533) et un ou plusieurs blocs de verrouillage (535) ;
la seconde partie de commande (520) inclut en outre une ou plusieurs parties de liaison en forme de dent (537) ; et
les un ou plusieurs boutons de verrouillage (533) sont configurés pour commander les un ou plusieurs blocs de verrouillage (535) afin de surmonter une force élastique du second élément élastique (531), de manière à libérer la restriction des un ou plusieurs blocs de verrouillage (535) sur les une ou plusieurs parties de liaison en forme de dent (537).

10. Poignée de commande (300) selon la revendication 1, comprenant en outre un mécanisme de commande à tube flexible (620) qui inclut une vis (621), une partie rotative (622) et une partie de traction (623), dans laquelle
la vis (621) et la partie de traction (623) sont reliées par filetage ;
la partie rotative (622) est configurée pour entraîner la vis (621) en rotation, entraînant ainsi le mouvement de la partie de traction (623) ; et
le mouvement de la partie de traction (623) commande la courbure d'un tube flexible (610).

11. Poignée de commande (300) selon la revendication 1, dans laquelle le mécanisme de commande de bras de serrage externe (400) inclut en outre un premier dispositif indicateur (810), le premier dispositif indicateur (810) étant configuré pour indiquer un angle d'ouverture du bras de serrage externe (220) en fonction d'une position d'au moins l'une de la première partie de commande (420) ou de la partie coulissante (430).

12. Poignée de commande (300) selon la revendication 11, dans laquelle le premier dispositif indicateur (810) inclut une ou plusieurs marques d'angle (811) disposées sur le manchon (410).

13. Poignée de commande (300) selon la revendication 11, dans laquelle
le premier dispositif indicateur (810) inclut un processeur (823), un dispositif d'affichage (825) et un premier capteur (821) ;
le premier capteur (821) est configuré pour détecter la position d'au moins l'une de la première partie de commande (420) ou de la partie coulissante (430) ; et
le processeur (823) est configuré pour déterminer l'angle d'ouverture du bras de serrage externe (220) en fonction de la position d'au moins l'une de la première partie de commande (420) ou de la partie coulissante (430), et commander au dispositif d'affichage (825) d'afficher l'angle d'ouverture du bras de serrage externe (220).

14. Poignée de commande (300) selon la revendication 1, dans laquelle le mécanisme de commande de bras de serrage interne (500) inclut en outre un second dispositif indicateur (910), le second dispositif indicateur (910) étant configuré pour indiquer un angle d'ouverture du bras de serrage interne (210) sur la base d'une position de la seconde partie de commande (520).

15. Dispositif de réparation de valvule mitrale (100), comprenant un dispositif de serrage de tissu (200) et une poignée de commande (300) selon l'une quelconque des revendications 1-14, dans lequel
le dispositif de serrage de tissu (200) inclut un bras de serrage externe (220) et un bras de serrage interne (210).
